# EUROPEAN PATENT SPECIFICATION

(11) **EP 3 964 247 B1**
(45) Date of publication and mention of the grant of the patent: **04.06.2025**
(21) Application number: 21178350.1
(22) Date of filing: 14.04.2017
(51) Int. Cl.: A61M 3/02

(54) **WEARABLE EAR CLEANING DEVICE**
AM KÖRPER TRAGBARER OHRREINIGER
DISPOSITIF DE NETTOYAGE DES OREILLES PORTABLE

(30) Priority: 30.06.2016 US 201662357320 P
(43) Date of publication of application: 09.03.2022
(62) Divisional of application: 17820700.7
(73) Proprietor: Safkan, Inc., Renton, Washington 98058 (US)
(72) Inventor: DIWAN, Aadil, Renton, 98058 (US)
(74) Representative: Cooley (UK) LLP

(56) References cited:
- US-A- 5 364 343
- US-A1- 2003 130 613
- US-A1- 2012 302 957
- US-B2- 8 840 546

## Description

### TECHNICAL FIELD

The present disclosure generally relates to a cannula.

### BACKGROUND

### Description of the Related Art

Ear wax, also known as cerumen, naturally forms in the outer portion of a person's auditory canal, and serves to protect and lubricate the auditory canal. The motion of a person's jaw assists in moving old ear wax towards the outside of the auditory canal where it dries up and falls away. Unfortunately, ear wax may build up in the auditory canal to the point that it impacts a person's hearing. Moreover, some people attempt to clean their ears with devices, such as cotton swabs, that generally push ear wax deeper into the auditory canal towards the ear drum. For this reason, attempting to clean the auditory canal with a cotton swab may actually harm a person's hearing without providing any substantial benefit.

US2012/302957 discloses an ear medication delivery device for delivering a treatment liquid and retaining the treatment liquid within an ear canal of a patient including a unitary, one piece earplug formed of a soft resilient material including an ear canal engaging portion and an external portion and defining a single internal passage therethrough. The external portion includes a major disk and an annular medication applicator receiving portion in fluid communication with the internal passage. The ear canal engaging portion includes at least two minor disks of progressively different size extending outwardly in a generally radial direction. The ear canal engaging portion defines a molded resilient valve unitary with the earplug that is shiftable from a closed position to an open position in either direction under a preselected fluid pressure and that resiliently returns to the closed position below the preselected fluid pressure.

US2003/130613 relates to a debridement extension providing irrigation and mechanical scrubbing for removal of dead, devitalized, or contaminated tissue from a wound.

US8,840546 discloses a system for accessing a body orifice.

US5,364,343 discloses an irrigation device for use in an ear canal, in which a main body of substantially conical shape has a central axis. There is at least one input duct connecting to a lavage liquid delivery apparatus for injecting the lavage liquid into the ear canal. There is at least one output duct allowing the continuous drainage of used lavage liquid. At least a part of the input duct has an axis which is inclined relative to the central axis of the main body. This directs impingement of the lavage liquid against wall surfaces and not against the eardrum.

### BRIEF SUMMARY

To clean the auditory canal, a mixture of saline, hydrogen peroxide, and/or water is used to irrigate the auditory canal and remove an excessive build-up of ear wax. Irrigating the auditory canal, however, may require a visit to a physician and the use of devices that require multiple people to operate. In addition, devices and techniques that are used to irrigate the auditory canal may result in used cleaning agent or cleaning fluid, usually carrying flushed ear wax, to exit the auditory canal and impact the patient.

In accordance with the invention, there is provided a cannula according to claim 1. Optional features are defined by the dependent claims.

### BRIEF DESCRIPTION OF THE SEVERAL VIEWS OF THE DRAWINGS

In the drawings, identical reference numbers identify similar elements or acts. The sizes and relative positions of elements in the drawings are not necessarily drawn to scale. For example, the shapes of various elements and angles are not necessarily drawn to scale, and some of these elements are arbitrarily enlarged and positioned to improve drawing legibility. Further, the particular shapes of the elements as drawn, are not necessarily intended to convey any information regarding the actual shape of the particular elements, and have been solely selected for ease of recognition in the drawings.
Figure 1A is an isometric view of an irrigation device.
Figure 1B is an isometric view of an over-ear earpiece.
Figure 2A is an exploded view of one of the over-ear earpieces of an irrigation device.
Figure 2B is an isometric view of an over-ear earpiece that has a rectangular form factor.
Figures 3A is an isometric view of a first side of a pan that may be included as a component of an over-ear earpiece.
Figures 3B is an isometric view of a second side of the pan of Figure 3A.
Figures 4 is an isometric view of a lid that may be included as a component of an over-ear earpiece.
Figures 5 is an isometric view of a cleaning agent reservoir of an over-ear earpiece.
Figures 6 is an isometric view of a discharge reservoir of an over-ear earpiece.
Figure 7A is an isometric view of a disposable cleaning agent reservoir of an over-ear earpiece.
Figure 7B is an isometric view of a disposable discharge collection reservoir of an over-ear earpiece.
Figure 7C is an isometric view of a shell broken into two cavities, one of which is sized and dimensioned to hold a disposable cleaning agent reservoir and the other of which is sized and dimensioned to hold a disposable discharge collection reservoir.
Figure 7D is an isometric view of a unitary disposable container that includes separate sections for a disposable cleaning agent reservoir and a disposable discharge collection reservoir.
Figure 8 is a side plan view of a cannula, according to one implementation, which may be used with or as part of an irrigation device.
Figure 9 is a front, side isometric view of the cannula of Figure 8, showing the tip from which cleaning agent exits and discharge enters the cannula.
Figure 10 is a rear, side isometric view of the cannula of Figure 8.
Figure 11 is a rear plan view of the cannula of Figure 8, which includes an interface that is used to couple the cannula to a cannula coupler interface on the over-ear earpieces.
Figure 12 is a transparent side, rear isometric view of the cannula of Figure 8, that shows the paths of an irrigation passage and a discharge collection passage within the interior of the cannula.
Figure 13 is a block diagram of controller circuitry and a power supply which may be used with or as part of an irrigation device.
Figure 14 shows an example of user-actable selectable controls.
Figure 15 provides a flow diagram of a set of user-actable selectable controls for operating an irrigation device.

### DETAILED DESCRIPTION

In the following description, certain specific details are set forth in order to provide a thorough understanding of various disclosed implementations. However, one skilled in the relevant art will recognize that implementations may be practiced without one or more of these specific details, or with other methods, components, materials, etc. In other instances, structures and solutions associated with ear cleaning, including the various components and ratios of such components in cleaning solutions, have not been shown or described in detail to avoid unnecessarily obscuring descriptions of the implementations.

Unless the context requires otherwise, throughout the specification and claims which follow, the word "comprise" and variations thereof, such as "comprises" and "comprising," are to be construed in an open, inclusive sense, that is, as "including, but not limited to."

Reference throughout this specification to "one implementation" or "an implementation" means that a particular feature, structure or characteristic described in connection with the implementation or implementations is included in at least one implementation or implementations. Thus, the appearances of the phrases "in one implementation" or "in an implementation" in various places throughout this specification are not necessarily all referring to the same implementation. Furthermore, the particular features, structures, or characteristics may be combined in any suitable manner in one or more implementation or implementations.

As used in this specification and the appended claims, the singular forms "a," "an," and "the" include plural referents unless the content clearly dictates otherwise. It should also be noted that the term "or" is generally employed in its sense including "and/or" unless the content clearly dictates otherwise.

The headings and Abstract of the Disclosure provided herein are for convenience only and do not interpret the scope or meaning of the implementations.

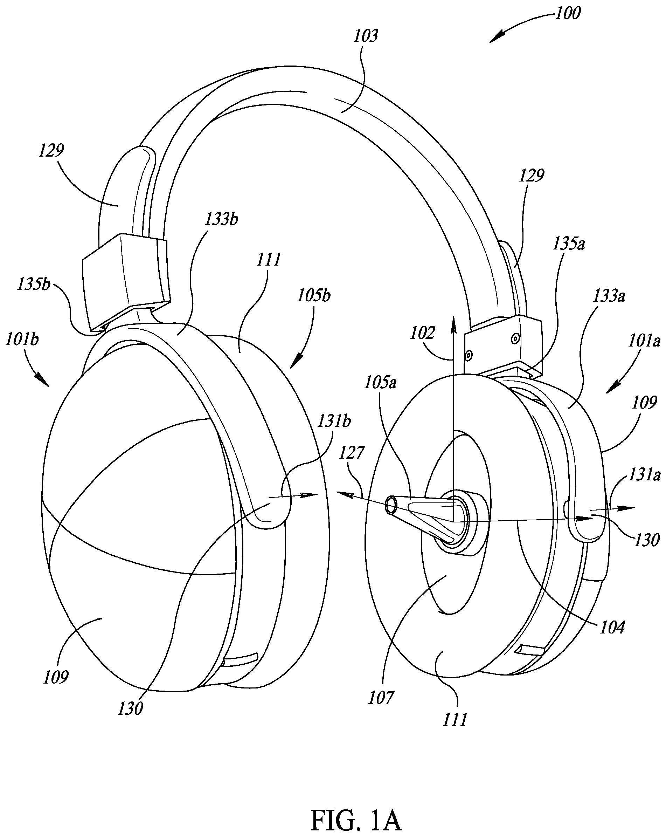

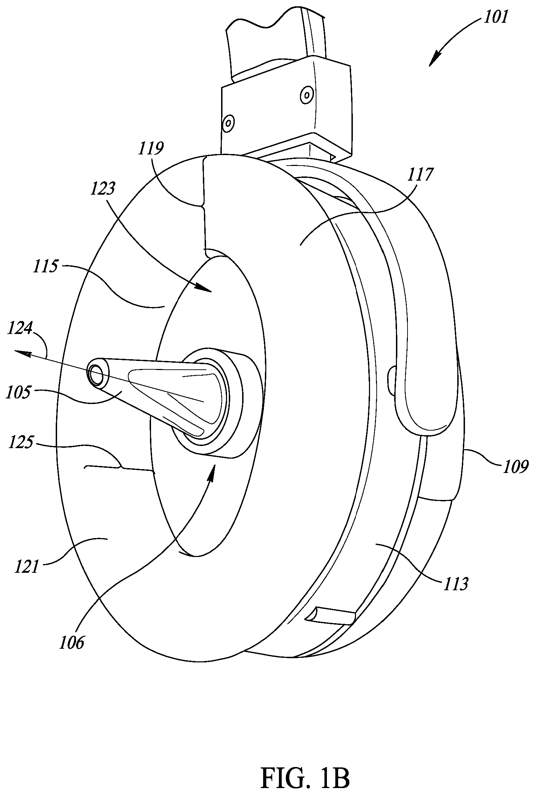

Figure 1A shows an irrigation device 100 that can be worn on a head of a human, according to at least one illustrated implementation not according to the invention. The irrigation device 100 includes a first and a second over-ear earpiece 101a, 101b (generically, "over-ear earpiece 101"), an adjustable head strap 103, and a first and a second cannula 105a, 105b (generically, "cannula 105") connected to respective cannula coupler interfaces 106a, 106b (only one visible in Figure 1A). Figure 1B shows the over-ear earpiece 101 in more detail.

In some implementations, the irrigation device 100 may be sized and shaped such that the first over-ear earpiece 101a fits over a user's right ear, and the second over-ear earpiece 101b fits over the user's left ear. Each over-ear earpiece may have a vertical axis 102 that is substantially perpendicular to the ground when a user wearing the irrigation device 100 is in an upright position, and a horizontal axis 104 that that is substantially parallel to the ground when the user wearing the irrigation device 100 is in the upright position is. While discussed with respect to an upright positon for ease of reference, it is noted that a user does not necessarily need to be in an upright position during use of the irrigation device 100.

Each over-ear earpiece 101 may include an interior side 107 and an opposing exterior side 109 in which the interior side 107 is directed towards and is adjacent the side of the user's head when the user wears the irrigation device 100. Each over-ear earpiece 101 may have a perimeter 113 that extends between the interior side 107 and the exterior side 109. The perimeter 113 may be sized and shaped so that the over-ear earpiece 101 can enclose a range of ear shapes. In some implementations, the perimeter 113 of the over-ear earpiece 101 may be substantially circular, oval, and/or elliptical in shape. In some implementations, the over-ear earpiece 101 may include a port 114 that may be used to provide power and/or communications to the irrigation device 100. The charging may be accomplished, for example, by connecting one or more physical connectors (*e.g.*, a USB connector) to thereby charge in internal battery within the irrigation device. In some implementations, the port 114 may enable inductive coupling to charge an internal battery. In some implementations, only one of the over-ear earpieces 101 may include a port 114. In some implementations, each of the over-ear earpieces 101 may be electrically coupled via, for example, one or more electrical connections running through the adjustable head strap 103.

The over-ear earpiece 101 may include an annular membrane 111 positioned on the interior side 107 at or proximate the perimeter 113 of the over-ear earpiece 101. In some implementations, the annular membrane 111 has an interior wall 115 and an exterior wall 117 that are separated by a distance 119 such that the interior wall 115 is concentric with, and completely enclosed by, the exterior wall 117. The interior wall 115 and the exterior wall 117 may be connected by a rim 121 that traverses the distance 119. In some implementations, the rim 121 may be a surface that curves outward, away from the over-ear earpiece 101, such as shown in Figure 1. In some implementations, the annular membrane 111 may be sized and shaped to resemble a portion of a solid torus (*e.g.*, the top half of a doughnut). Such a curved or partially toroidal surface may be used to increase the user's comfort when wearing the over-ear earpiece 101. In some implementations, the rim 121 may form a substantially flat surface that is perpendicular to one or both of the interior wall 115 and the exterior wall 117.

In some implementations, the annular membrane 111 may be made of cushioned material that compresses when a force is applied against it. Such material may include, for example, an elastomer, closed-cell foam (*e.g.*, polyurethane), open cell foam, gel in a pouch, silicone, and/or rubber which may include an outer cover of plastic, leather, or leatherette material. In some implementations, the annular membrane 111 may be comprised of resilient material to increase its longevity and durability. Such cushioned and/or resilient material may be used to provide a more comfortable fit for the user when wearing the irrigation device 100. In some implementations, the cushioned material may conform to the shape of the side of the user's head when the irrigation device 100 is in use. As such, the cushioned material may thereby form a seal against the user's head to trap cleaning agent that escapes from the cannula 105 and/or the user's auditory canal during an irrigation procedure. The annular membrane 111 may be comprised, for example, of elastomer-based materials. In some implementations, the annular membrane 111 may be formed of a core composed at least in part of elastomer-based materials and a corresponding cover that may be composed of material that protects the core from the surrounding environment, such as a plastic or other synthetic material that does not degrade when wet. In some implementations, the annular membrane 111 may be detachably removed from the over-ear earpiece 101, and thus may be replaced after each use of the irrigation device 100.

The interior wall 115 of the annular membrane 111 forms a cavity 123 that is sized and shaped to enclose and receive one of the user's ears. The size and shape of the annular membrane 111 and the cavity 123 may further be used to position the cannula 105 within the user's auditory canal when the user is wearing the irrigation device 100. In some implementations, the cavity 123 may be cylindrical in shape and have the cannula 105 oriented along a central axis 124 of the cavity 123. In some implementations, the cavity 123 may be elongated such that the perimeter of the interior wall 115 forms a substantially elliptical or oval shape. In such an implementation, the cavity may be relatively longer along the vertical axis 102, and relatively shorter along the horizontal axis 104. Elongating the cavity 123 in such a way may provide for a better, more comfortable fit for the user. The cavity 123 may further be sized and dimensioned to receive ears of various sizes.

The cavity 123 may have a depth formed by a height 125 of the interior wall 115. In some implementations, the depth of the cavity 123 may be less than a height of the cannula 105, enabling the cannula 105 to extend past the rim 121 of the annular membrane 111 and thereby enter the user's auditory canal when the user wears the irrigation device 100. In some implementations, as discussed below, the cannula 105 may further be tapered, with a tip that is narrower than a base of the cannula 105, to assist in inserting and positioning the tip of the cannula 105 into the user's auditory canal. The tapering of the cannula 105, however, can be designed such that a portion of the cannula 105 between the tip and the base impacts the outside opening of the user's auditory canal before the tip of the cannula 105 impacts the user's ear drum, thereby preventing injury to the ear drum.

In some implementations, the irrigation device 100 includes an adjustable head strap 103 that adjustably connects the first over-ear earpiece 101a and the second over-ear earpiece 101b in a spaced apart relation along a lateral axis 127 that extends between the two opposing cannula coupler interfaces 106a and 106b. The adjustable head strap 103 may be sized and shaped to fit over the user's head, thereby concurrently positioning the first over-ear earpiece 101a over a user's first ear and the second over-ear earpiece 101b over the user's second ear. The adjustable head strap 103 may be flexible such that the first over-ear earpiece 101a and the second over-ear earpiece 101b may be moved or flexed laterally along the lateral axis 127. In some implementations, one or both of the over-ear earpieces 101a, 101b may include vertical adjustors 129 that allow the over-ear earpieces 101 to move along the vertical axis 102 with respect to the adjustable head strap 103 when a user is wearing the irrigation device 100. The vertical adjustors 129 may be used, for example, to adjust the irrigation device to accommodate different circumferential distances between various users' ears (*e.g.*, from one ear of a user, over the top of the user's head, and to the other ear of the user). Thus, one or both of the vertical adjustors 129 may be extended to accommodate users having relatively larger heads.

The adjustable head strap 103 may connect to each of the over-ear earpieces 101 via one or more pivot couplers 130. The pivot couplers 130 may enable each of the over-ear earpieces 101a, 101b to independently pivot around respective axes 131a, 131b that run between the pivot couplers 130 on each respective over-ear earpieces 101a, 101b; the respective axes 131a, 131b may be perpendicular to the lateral axis 127 that extends between the first cannula coupler interface 106a and the second cannula coupler interface 106b. Such pivot couplers 130 enable each of the over-ear earpieces 101a, 101b to more comfortably fit the contours and shape of a user's head when the user operates the irrigation device 100.

In some implementations, the over-ear earpieces 101a, 101b may include respective annular brackets 133a and 133b. The annular brackets 133a, 133b may be sized and shaped to be slightly larger than a portion of the perimeter 113 of the respective over-ear earpiece 101a, 101b. For example, the annular brackets 133a, 133b may be sized and shaped to run along the top half of the perimeters 113 of the respective over-ear earpieces 101a, 101b. In such implementations, the annular brackets 133a, 133b may be pivotally coupled to the adjustable head strap 103 at junctions 135a and 135b, thus enabling each of the over-ear earpieces 101 to independently pivot relative to the junctions 135a and 135b.

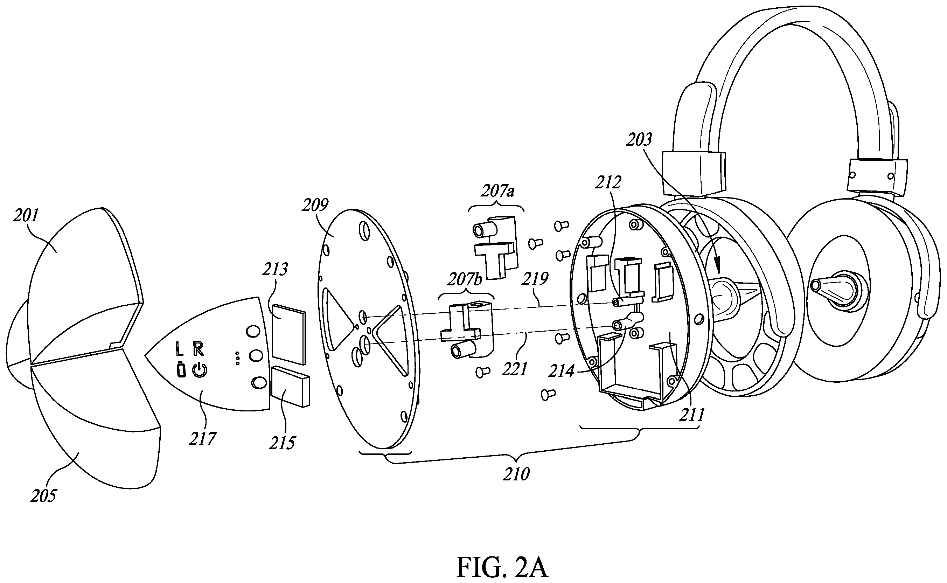

Figure 2A shows the components of one of the over-ear earpieces 101 of the irrigation device 100, according to at least one illustrated implementation not in accordance with the invention. The components of the over-ear earpiece 101 may include a cleaning agent reservoir 201, a cannula coupler interface 203, and a discharge
collection reservoir 205. Components of the over-ear earpiece 101 may also include a pump assembly 207a that may have a pressure pump and/or manifold to couple to the cleaning agent reservoir 201 and operable to generate a positive pressure (*e.g.*, greater than one atmosphere or greater than ambient pressure) and a vacuum assembly 207b that may have a vacuum pump and/or manifold to couple to the discharge collection reservoir 205 and operable to generate a negative pressure (*e.g.*, less than one atmosphere or less than ambient pressure), a housing 210 that includes a lid 209 and a pan 211, controller circuitry 213, one or more power supplies 215, and a set of user-actable selectable controls 217.

The cleaning agent reservoir 201 holds cleaning agents that are to be used to clean the user's auditory canal. Such a cleaning agent may include, for example, one or more of saline, hydrogen peroxide, and/or water. In some implementations, the cleaning agent reservoir 201 may be filled with cleaning agent that is heated to a temperature at, or slightly above or below, the user's body temperature for the user's comfort. In some implementations, the cleaning agent may be reservoir 201 may be filled with cleaning agent at or around body temperature or slightly warmer. In some implementations, the cleaning agent reservoir 201 may include or be physically coupled to a heating component (*e.g.*, heater or resistive element) positioned to heat the contained cleaning agent to and/or maintain the contained cleaning agent at a temperature at or around the user's body temperature. Such a heating component may be electrically and communicatively coupled to the controller circuitry 213, which may transmit control signals to the heating component. In some implementations, the cleaning agent reservoir 201 may include an internal thermometer to measure the temperature of the cleaning agent contained within the cleaning agent reservoir 201. In such an implementation, the thermometer may by electrically and communicatively coupled to the controller circuitry 213. In such an implementation, the thermometer may transmit to the controller circuitry 213 electrical signals indicative of the temperature within the cleaning agent reservoir 201. In some implementations, the controller circuitry 213 may use the signals from the thermometer as feedback to control the heating component. In some implementations, the controller circuitry 213 may use the signals from the thermometer to provide for the safety and/or comfort of the user by preventing an irrigation procedure from being performed when the cleaning agent held within the cleaning agent reservoir 201 is either too hot or too cold.

The cleaning agent reservoir 201 may be in fluid communication with the cannula coupler interface 203 using a cleaning fluid fluidly communicative path 219. As shown in Figure 2A, the cleaning fluid fluidly communicative path 219 may proceed through an opening in the lid 209, through a cleaning agent passage 212 in the housing 210, to the cannula coupler interface 203. When an irrigation procedure begins, cleaning agent exits the cleaning agent reservoir 201 and proceeds through the cleaning fluid fluidly communicative path 219 to the cannula 105. Air or liquid pressure from the pump assembly 207a may be applied to the cleaning agent reservoir 201 to facilitate the flow of the cleaning agent from the cleaning agent reservoir 201. In some implementations, the pump assembly 207a may be a water or liquid pump that is used to draw the cleaning agent from the cleaning agent reservoir 201 and discharge the cleaning agent towards the cannula 105 at a desired positive pressure. The cleaning agent may proceed into the cannula to be discharged through one or more irrigation outlet apertures, as discussed further below.

In some implementations, the irrigation device 100 may leave the cleaning agent in the user's auditory canal for a period of time (*e.g.*, at least one minute) before removing the cleaning agent. In some implementations, this time period may be set by the user via the user-actable selectable controls 217. In some implementations, the controller circuitry 213 may store processor-executable instructions that define one or more pre-set time periods for the cleaning agent to remain in the user's auditory canal. The user may choose one of these pre-set time periods via the user-actable selectable controls 217 such that the associated processor-executable instructions are executed by a processor included in the controller circuitry 213. This time period between introducing the cleaning agent into and removing it from the auditory canal may enable the cleaning agent to further soften buildup in the user's auditory canal and thus enhance the cleaning capabilities of the irrigation device 100 and the user's experience.

When the time period ends, the discharge from the irrigation is removed from the user's auditory canal via a discharge collection inlet port located on the cannula 105, as discussed below. The discharge exits the cannula 105 to a discharge fluidly communicative path 221 that proceeds from the cannula coupler interface 203, through a discharge passage 214 in the housing 210, and through one or more openings in the lid 209, to the discharge collection reservoir 205. In some implementations, the vacuum assembly or vacuum pump 207b is used to create a vacuum or area of low pressure (*i.e.*, lower than ambient environmental pressure, e.g., lower than 1 atmosphere) within the discharge collection reservoir 205 to facilitate the removal of the discharge from the auditory canal. References to vacuum herein and in the claims refer to a pressure that is lower than ambient environmental pressure (e.g., lower than 1 atmosphere) rather than an absolute vacuum.

In some implementations, the irrigation device 100 may pulse cleaning agent into the user's auditory canal for a period of time (*e.g.*, up to 30 seconds, 35 seconds, up to a one minute, or longer). Such pulses may occur, for example, every couple of seconds. In such an implementation, the vacuum assembly 207b may be actuated before the cleaning agent is first pulsed into the user's auditory canal and remain actuated during the entire time period. The vacuum assembly 207b may be deactivated after the time period ends.

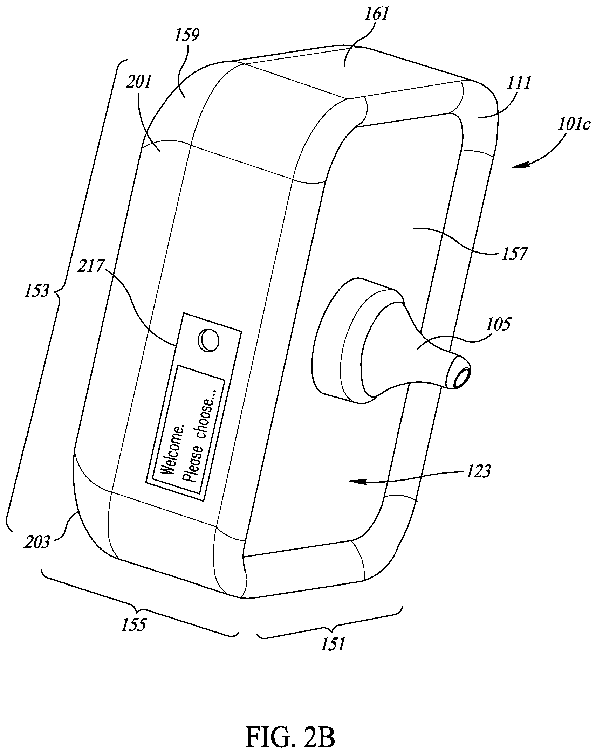

Figure 2B shows an implementation of an irrigation device 100 with a rectangular over-ear earpiece 101c, according to one illustrated implementation not in accordance with the invention. Such a rectangular over-ear earpiece 101c may have a width 151, a height 153, and a depth 155 with an interior side 157 and an exterior side 159 that are connected by one or more side walls 161. In some implementations, the depth 155 may be less than the width 151, and the width 151 may be less than the height 153. Such dimensions may provide a more streamlined fit and appearance when compared to over-ear earpieces 101 of other shapes. When placed over an ear of a user, the height 153 may be oriented vertically with the interior side 157 adjacent to the side of the face of the user, perpendicular to the ground when the user wearing the irrigation device 100 is in an upright position. The cannula 105 may be located substantially in the center of the interior side 157 of the rectangular over-ear earpiece 101c to position the cannula 105 within the auditory canal of the user when the user is wearing the irrigation device 100 with the rectangular over-ear earpiece 101c. The rectangular over-ear earpiece 101c may include an annular membrane 111 that extends around the perimeter of the interior side 157. As discussed above, the annular membrane may form a cavity 123 that may be sized and dimensioned to receive ears of various sizes. The cleaning agent reservoir 201 and a discharge collection reservoir 205 may be enclosed within one or more containers attached to the rectangular over-ear earpiece 101c along the exterior side 159. A set of set of user-actable selectable controls 217 may be located along one of the side walls 161. In some implementations, the set of user-actable selectable controls 217 may include one or more buttons, switches, and/or a touch screen pad for entering commands to, and receiving status updates and feedback from, the irrigation device 100.

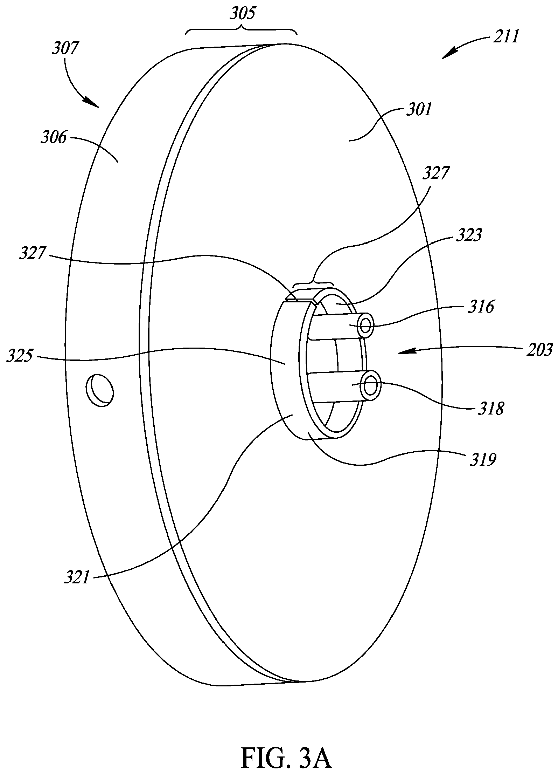

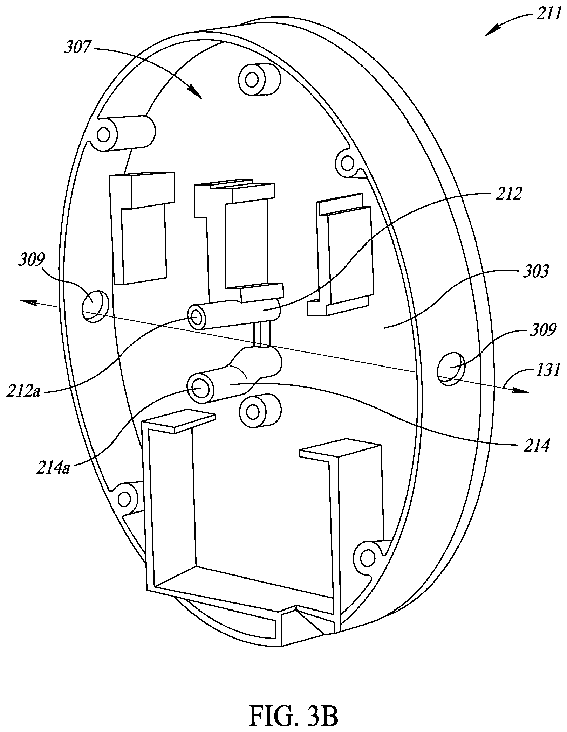

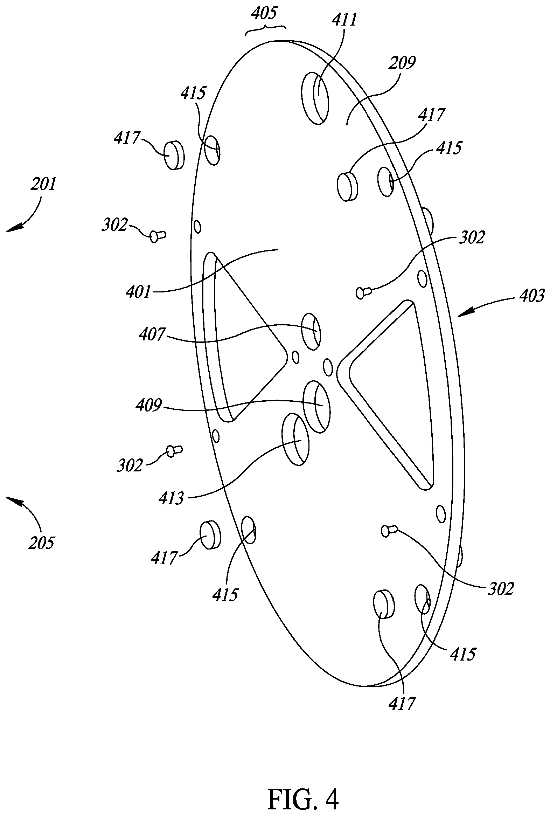

Figures 3A and 3B are isometric views of the a first side 301 and a second side 303 of the pan 211, respectively, and Figure 4 is an isometric view of the lid 209, according to at least one illustrated implementation not in accordance with the invention. As previously noted, the pan 211 and the lid 209 may together form the housing 210. The first side 301 of the pan 211 faces the interior side 107 of the over-ear earpiece 101 towards the cannula 105. The second side 303 of the pan 211 faces the exterior side 109 of the over-ear earpiece 101 towards the cleaning agent reservoir 201 and the discharge collection reservoir 205. The lid 209 attaches to the second side 303 of the pan 211 via one or more coupling features, *e.g.*, screws 302. The lid 209 (when attached to the pan 211) and the first side 301 of the pan 211 are separated by a distance 305 that forms the depth of the housing 210. A side wall 306 bridges the distance 305 between the lid 209 and the first side 301 of the pan 211. The attached lid 209, the first side 301 of the pan 211, and the side wall 306 define an interior 307 of the housing 210.

As shown in Figure 3A, the cannula coupler interface 203 may include a cleaning agent port 316, a vacuum port 318, and one or more interfaces 319. The cleaning agent port 316 may be part of the cleaning fluid fluidly communicative path 219 for the cleaning agent and may mate with a corresponding irrigation inlet port located on the cannula 105. For example, the cleaning agent port 316 may be a male connector or fastener, and the irrigation inlet port located on the cannula 105 may be a corresponding female connector or fastener. Mating the cleaning agent port 316 with the irrigation inlet port may be used to form part of a fluid path for the cleaning agent to travel from the cleaning agent reservoir 201 to the cannula 105. The vacuum port 318 may be part of the discharge fluidly communicative path 221 for the discharge and may mate with a corresponding discharge collection outlet port located on the cannula 105. For example, the vacuum port 318 may be a male connector or fastener, and the discharge collection outlet port located on the cannula 105 may be a corresponding female connector or fastener. Mating the vacuum port 318 with the discharge collection outlet port may be used to form part of a fluid path for the discharge to travel from the cannula 105 to the discharge collection reservoir 205.

The cannula coupler interface 203 may have one or more interfaces 319 that physically couple to one or more corresponding interfaces on the cannula 105. These complementary interfaces may enable the cannula 105 to be selectively detachable and physically coupled to the cannula coupler interface 203 on the over-ear earpieces 101. In some implementations, the interface 319 for the cannula coupler interface 203 may be in the form of an annular wall 321 with an interior diameter 323, an exterior diameter 325, and a height 327. The interior diameter 323 and the exterior diameter 325 may form concentric shapes (*e.g.*, circles, ovals, squares, *etc*.) that encompass the cleaning agent port 316 and the vacuum port 318. The annular wall 321 may physically engage and be coupled with a corresponding interface on the cannula 105, such as, for example, a slot or opening that is sized and shaped to be only slightly larger than the annular wall 321. As such, the frictional forces may keep the cannula 105 physically coupled to the cannula coupler interface 203. In such an implementation, one or more flexible, compressible features, such as an O-ring, may be used to form an air-tight and/or water-tight seal between the coupling interfaces on the cannula coupler interface 203 and the cannula 105. In some implementations, the interface 319 may have one or more registration features that serve to properly align the cleaning agent port 316 and the vacuum port 318 of the cannula coupler interface 203 with the corresponding irrigation inlet port and discharge collection outlet port located on the cannula 105 when the interface 319 is engaged with the corresponding interface on the cannula 105. For example, the annular wall 321 may have a slot 329 that is sized and shaped to receive a corresponding tab that extends from the corresponding interface on the cannula 105. Aligning the slot 329 and the tab may be used to ensure that the cleaning agent port 316 and the vacuum port 318 of the cannula coupler interface 203 are properly aligned with the irrigation inlet port and discharge collection outlet port, respectively, located on the cannula 105.

In some implementations, the cannula coupler interface 203 may include other or additional types of interfaces 319 to physically detachably couple with the cannula 105. For example, the cannula coupler interface 203 and the cannula 105 may include complementary threads that enable the cannula 105 to be screwed onto the cannula coupler interface 203. In some implementations, the cannula 105 may be attached to the cannula coupler interface 203 using a bayonet-style coupler. In some implementations, the cannula 105 is sized and shaped to fit tightly over and couple to the cannula coupler interface 203 using one or more latches or other coupling features.

Figure 3B is an isometric view of a second side 303 of the pan 211, which may include a cleaning agent passage 212 and a discharge passage 214. The cleaning agent passage 212 may provide a first passage through the housing 210 from the cleaning agent reservoir 201 to the cannula coupler interface 203, thereby forming part of the cleaning fluid fluidly communicative path 219. The opening 212a of the cleaning agent passage 212 directed towards the cleaning agent reservoir 201 may be size, shaped, and positioned to mate with a port on the cleaning agent reservoir 201. For example, the opening 212a may form a male connector or fastener that mates with a female connector or fastener that forms a port on the cleaning agent reservoir 201. The pan 211 may include a discharge passage 214 that provides a second passage through the housing 210 from the cannula coupler interface 203 to the discharge collection reservoir 205, thereby forming part of the discharge fluidly communicative path 221. The opening 214a of the discharge passage 214 directed towards the discharge collection reservoir 205 may be size, shaped, and positioned to mate with a port on the discharge collection reservoir 205. For example, the opening 214a may form a male connector or fastener that mates with a female connector or fastener that forms a port on the discharge collection reservoir 205. In some implementations, either or both of the cleaning agent passage 212 and the discharge passage 214 may be part of the cannula 105. In such an implementation, each cannula 105 will provides its own cleaning agent passage 212 to the cleaning agent reservoir 201 and/or its own discharge passage 214 to the discharge collection reservoir 205.

In some implementations, one or both of the cleaning agent passage 212 and the discharge passage 214 are sealed from the remaining part of the interior 307 of the housing 210. Thus, the cleaning agent and the discharge proceed through the cleaning agent passage 212 and the discharge passage 214, respectively, without leaking into the interior 307 of the housing 210. In some implementations, the interior 307 of the housing 210 may enclose and be used to secure one or more additional components, such as for example the pressure pump assembly 207a, the vacuum pump assembly 207b, the controller circuitry 213, and/or the power supplies 215.

The pan 211 may include one or more coupling apertures 309 located on the side wall 306 to enable the pan 211 and/or the housing 210 to be coupled to the annular bracket 133. In some implementations, for example, the annular bracket 133 may have complementary coupling features *(e.g.,* tabs) that correspond to the one or more coupling apertures 309 located on the side wall 306. As such, the housing 210, and by extension the over-ear earpieces 101, may be selectively detachably coupled to the annular bracket 133 via the corresponding coupling features on the pan 211 and the annular bracket 133. The coupling features on the annular bracket 133 and the corresponding coupling apertures 309 in the housing 210 may form a set of pivot couplers 130 that, as discussed above, enable the over-ear earpieces 101 to pivot relative to the axis 131 that runs between the coupling features.

Figure 4 shows the lid 209, which has a first side 401 and an opposing second side 403 separated by a distance 405, according to at least one illustrated implementation not in accordance with the invention. The first side faces the exterior side 109 of the 20 over-ear earpiece 101 towards the cleaning agent reservoir 201 and the discharge collection reservoir 205. The second side 403 faces the interior side 107 of the over-ear earpiece 101 towards the pan 211 and the cannula coupler interface 203. The lid 209 may include a cleaning agent aperture 407 that enables the cleaning agent to pass from the cleaning agent reservoir 201 to the cannula 105. The lid 209 may include a discharge aperture 409 that enables the discharge to pass from the cannula 105 to the discharge collection reservoir 205. The lid 209 may include a pump aperture 411 that enables the output of the pump assembly 207a, located on the second side 403 of the lid 209, to be physically coupled to the cleaning agent reservoir 201, located on the first side 401 of the lid 209. The pump assembly 207a may include a pressure pump and/or manifold operable to generate a positive pressure *(e.g.,* greater than one atmosphere or greater than ambient pressure) within the cleaning agent reservoir 201. As shown in Figure 4, the pump aperture 411 may be located proximate the edge of the lid 209. The lid 209 may include a vacuum aperture 413 that enables the vacuum assembly 207b, located on the second side 403 of the lid 209, to be physically mated with the discharge collection reservoir 205, located on the first side 401 of the lid 209. The vacuum assembly 207b may include a vacuum pump and/or manifold operable to generate a negative pressure *(e.g.,* less than one atmosphere or less than ambient pressure) within the discharge collection reservoir 205 to draw discharge from the cannula 105 out of the auditory canal and into the discharge collection reservoir 205.

The lid 209 may include one or more coupling features 415. The coupling features 415 may be used to secure one or both of the cleaning agent reservoir 201 and the discharge collection reservoir 205 to the lid 209. In some implementations, the coupling features 415 may be magnets and/or ferromagnetic pieces, such as magnets 417, that have corresponding magnets of opposite polarity in either the cleaning agent reservoir 201 or the discharge collection reservoir 205. The use of magnets 417 as the coupling feature 415 may advantageously enable a user to quickly and easily remove the cleaning agent reservoir 201 for filling and/or the discharge collection reservoir 205 for emptying while also ensuring that both the cleaning agent reservoir 201 and the discharge collection reservoir 205 can be securely coupled to the over-ear earpiece 101.

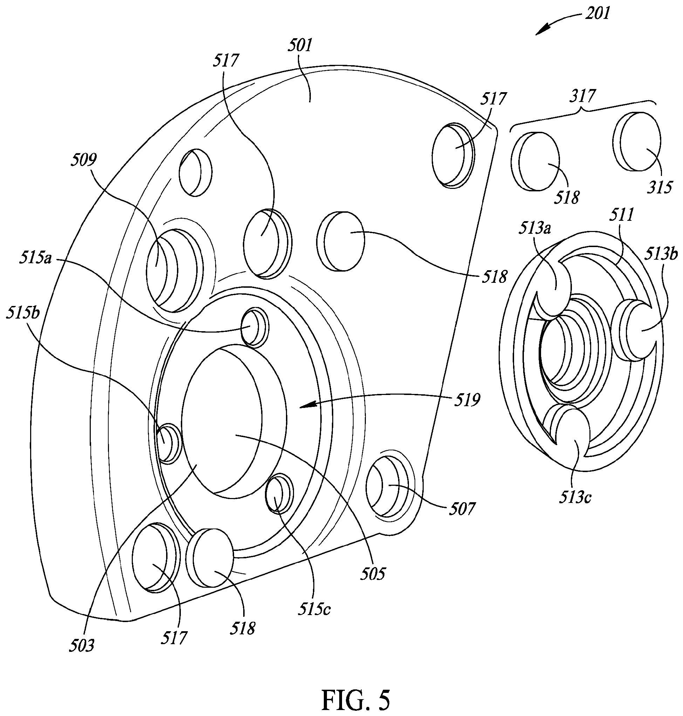

Figure 5 is an isometric view of the cleaning agent reservoir 201, according to at least one illustrated implementation not according to the invention. The cleaning agent reservoir 201 may include a shell 501 having a plurality of sides that define an enclosed space 503 in which the cleaning agent is held. In some implementations, the enclosed space 503 may have a volume that holds sufficient cleaning agent for performing one or more irrigation procedures. In some implementations, for example, the enclosed space 503 may hold up to 100 mL of cleaning agent. The shell 501 may include multiple apertures, including a loading aperture 505, a cleaning agent port 507, and a pressure aperture 509.

The loading aperture 505 may be used to load the cleaning agent into the enclosed space 503. In some implementations, the loading aperture 505 may be mated with and sealed by a cleaning agent cover 511. In some implementations, the cover 511 may include one or more magnets 513a, 513b, and 513c that magnetically couple to corresponding magnets 515a, 515b, and 515c of the opposite polarity, thus providing multiple pairs of ferromagnetic couplers spaced proximate the edge of the loading aperture 505. Alternatively or in addition, in some implementations, the loading aperture 505 and the cleaning agent cover 511 may have complementary threads that enable the cleaning agent cover 511 to be screwed into the loading aperture 505. In some implementations, the cover 511 may include a protrusion that is sized and shaped to fit tightly with a corresponding slot or opening proximate the edge of the loading aperture 505. In some implementations, such a protrusion for the cover 511 may be sized and shaped to fit tightly inside the loading aperture 505 itself. A flexible, water-tight material (*e.g.*, an O-ring) may be placed to go around the portion of the cover 511 that is adjacent to the edge of the loading aperture 505 for a water-tight and air-tight seal. As such, in some implementations, the cleaning agent reservoir 201 may be formed by the shell 501 and the cleaning agent cover 511.

In some implementations, the cleaning agent port 507 is sized and shaped to mate with the opening 212a of the cleaning agent passage 212 that extends through the housing 210, thus providing an exit for the cleaning agent from the enclosed space 503. In some implementations, the pressure aperture 509 is mated to an output of the pump assembly 207a. In such implementations, the pump assembly 207a may be used to increase the pressure inside the enclosed space 503, thus forcing the cleaning agent through the cleaning agent port 507 at increased pressure. In some implementations, the pump assembly 207a may be a water or liquid pump that draws cleaning agent directly from the cleaning agent port 507 and dispenses it through the cleaning agent passage 212 at a specified positive pressure. In such an implementation, the cleaning agent reservoir 201 may not include the pressure aperture 509. The pressure for the pump assembly 207a may be set so as to not cause discomfort to the user when the cleaning agent exits the cannula 105. In some implementations, for example, the pressure for the pump assembly 207a may be less than about 100 kPa.

The shell 501 may include one or more coupling features 517 that couple with the corresponding coupling features 311 located on the lid 209 and or the housing 210, thereby securing the shell 501 to the housing 210 as discussed above. In some implementations, the coupling features 517 may include magnets 518 that magnetically couple with the corresponding magnets 315 of opposite polarity located on the housing 210. Each magnetic pair may thereby form a pair of ferromagnetic couplers 317. The ferromagnetic couplers 317 may be used to selectively, detachably, magnetically couple the shell 501 and the cleaning agent reservoir 201 to the housing 210.

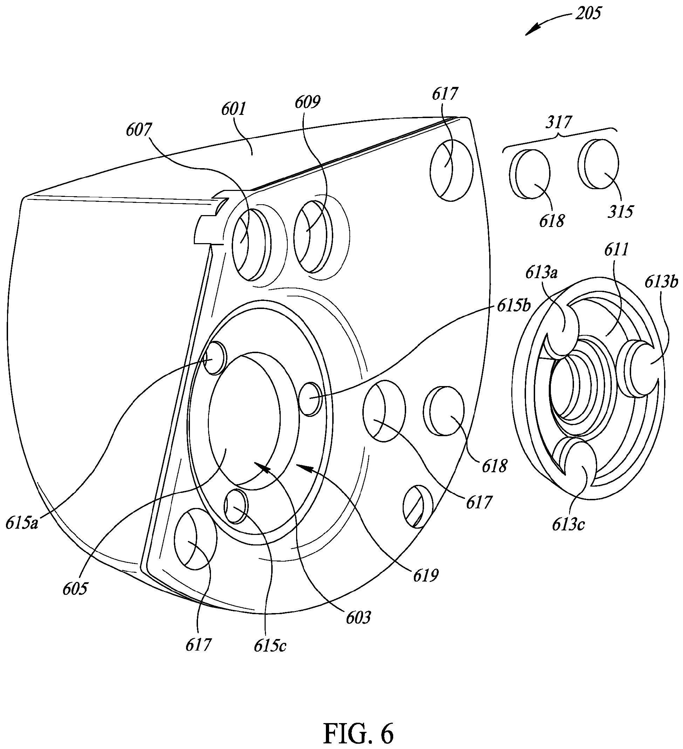

In some implementations, the loading aperture 505 may be inside a depression or well 519 placed into the shell 501. Accordingly, when the cleaning agent cover 511 is secured in the shell 501, the cleaning agent cover 511 may be at or below a plane formed by the side of the shell 501 to which the cleaning agent cover 511 attaches. As such, the cover 511 may not impede or interfere with the magnetic coupling of the ferromagnetic couplers 317. Figure 6 is an isometric view of the discharge collection reservoir 205, according to at least one illustrated implementation not according to the invention. As shown in Figure 6,
the discharge collection reservoir 205 may include a shell 601 having a plurality of sides that define an enclosed space 603 in which the discharge from the irrigation is held. In some implementations, the enclosed space 603 may have a volume that is slightly larger than the volume of cleaning agent held by the cleaning agent reservoir 201. The shell 601 may include multiple apertures, including a discharge removal aperture 605, a discharge port 607, and a vacuum aperture 609.

The discharge removal aperture 605 may be used to remove the discharge from the enclosed space 603. In some implementations, the discharge removal aperture 605 may be mated with and sealed by a discharge cover 611. In some implementations, for example, the discharge cover 611 may include one or more magnets 613a, 613b, and 613c that magnetically couple to corresponding magnets 615a, 615b, and 615c of the opposite polarity, thus providing multiple pairs of ferromagnetic couplers spaced proximate the edge of the discharge removal aperture 605. Alternatively or in addition, the discharge removal aperture 605 and the discharge cover 611 may have complementary threads that enable the discharge cover 611 to be screwed into the discharge removal aperture 605. In some implementations, the discharge cover 611 may include a protrusion that is sized and shaped to fit tightly with a corresponding slot or opening proximate to the edge of the discharge removal aperture 605. In some implementations, such a protrusion for the discharge cover 611 may be sized and shaped to fit tightly inside the discharge removal aperture 605 itself. A flexible, water-tight material (e.g., an elastomeric O-ring) may go around the portion of the discharge cover 611 that is adjacent to the edge of the discharge removal aperture 605 to form a water-tight and air-tight seal. As such, in some implementations, the discharge collection reservoir 205 may be formed by the shell 601 and the discharge cover 611.

In some implementations, the discharge port 607 is sized and shaped to mate with the opening 214a of the discharge passage 214 that extends through the housing 210, thus providing an exit for the discharge to travel to the enclosed space 603. In some implementations, the vacuum aperture 609 is mated to an output of the vacuum assembly 207b. In such implementations, the vacuum assembly 207b may be used to form an area of low pressure inside the enclosed space 603, thus drawing discharge from the discharge port 607 into the enclosed space 603. The vacuum pressure for the vacuum assembly 207b may be set for the comfort and/or safety of the user. In some implementations, for example, the vacuum pressure for the vacuum assembly 207b may be less than about 100 kPa.

The shell 601 may include one or more coupling features 617 that couple with corresponding coupling features 311 located on the housing 210, thereby securing the shell 601 to the housing 210 as discussed above. In some implementations, the coupling features 617 may include magnets 618 that magnetically couple with corresponding magnets 315 of opposite polarity located on the housing 210, thereby forming a pair of ferromagnetic couplers 317. The ferromagnetic couplers 317 may be used to detachably, magnetically couple the shell 601 and the discharge collection reservoir 205 to the housing 210. Further, in some implementations, the shell 501 for the cleaning agent and the shell 601 for the discharge may be independently coupled to the housing 210. In such implementations, each of the cleaning agent shell 501 and the discharge shell 601 may be selectively and independently detached from the housing 210 with respect to the other.

In some implementations, the removal aperture 605 may be inside a depression or well 619 placed into the shell 601. Accordingly, when the discharge cover 611 is secured in the shell 601, it may be at or below a plane formed by the side of the shell 601 to which the discharge cover 611 attaches. As such, the discharge cover 611 may not impede or interfere with the magnetic coupling of the ferromagnetic couplers 317.

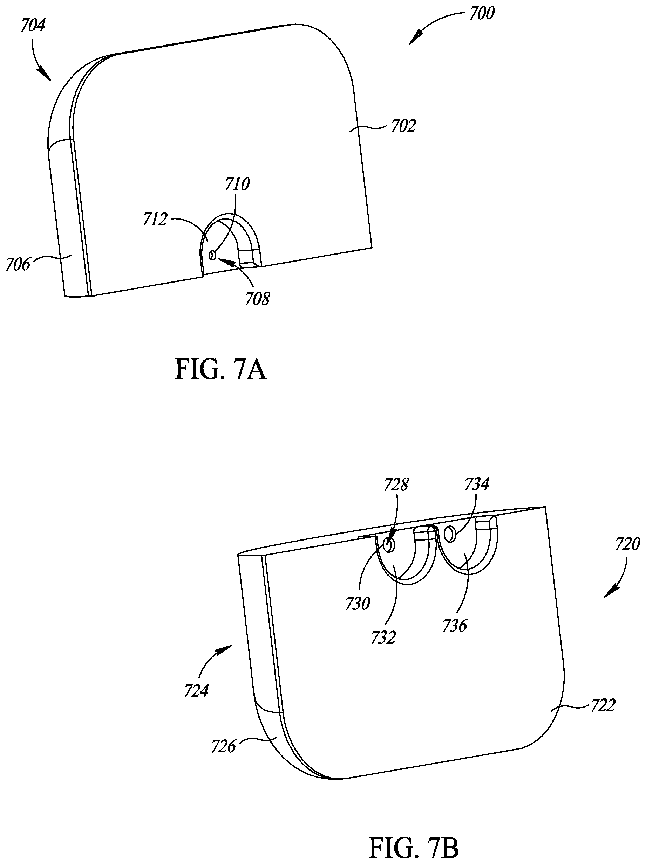

Figure 7A is an isometric view of a disposable cleaning agent reservoir 700 of an over-ear earpiece 101, according to at least one illustrated implementation not according to the invention. The disposable cleaning agent reservoir 700 may include a front face 702 and a back face 704. In some implementations, the front face 702 and the back face 704 may be substantially parallel to each other. In such implementations, the disposable cleaning agent reservoir 700 may include the one or more side walls 706 that bridge the distance between the front face 702 and the back face 704. In some implementations, the front face 702 and the back face 704 may meet at an edge such that the back face 704 curves outward from the front face 702 and/or the front face 702 curves outward from the back face 702. In such an implementation, a cross-sectional area of the disposable cleaning agent reservoir 700 may include an arc along one or both of the sides associated with the back face 704 and the front face 702.

The front face 702, the back face 704, and the side walls 706 (when present) may define a hollow space 708 for the disposable cleaning agent reservoir 700, with each of the front face 702, the back face 704, and the side walls 706 delineating a boundary for the hollow space. In some implementations, the hollow space 708 may be accessible via a disposable cleaning agent reservoir port 710 located within a depression 712 located on the front face 702 of the disposable cleaning agent reservoir 700. The disposable cleaning agent reservoir port 710 may be sized and dimensioned to selectively, detachably, physically mate with the cleaning fluid fluidly communicative path 219 at an end opposite the cannula 105, e.g., at the opening 212a of the cleaning agent passage 212. When the disposable cleaning agent reservoir port 710 is mated with the cleaning fluid fluidly communicative path 219, the disposable cleaning agent reservoir 700 may provide cleaning agent for one or more irrigation procedures. In some implementations, the disposable cleaning agent reservoir port 710 may be fluidly coupled to a pump assembly 207a that is a water pump that draws cleaning agent out of the disposable cleaning agent reservoir 700 towards the cannula 105. In some implementations, the disposable cleaning agent reservoir port 710 may be sealed with a flexible membrane during a manufacturing and/or assembly process after cleaning agent has been placed into the hollow space 708 of the disposable cleaning agent reservoir 700. In some implementations, the cleaning fluid fluidly communicative path 219 (*e.g*., the tip of the cleaning agent passage 212) may puncture the membrane thereby accessing the cleaning fluid when the disposable cleaning agent reservoir 700 is mounted onto an over-ear earpiece 101.

The disposable cleaning agent reservoir 700 may be made of any suitable plastic materials, such as, for example, plastic material that is approved for use in medical procedures. In some implementations, the disposable cleaning agent reservoir 700 may hold sufficient cleaning agent to perform one (1) irrigation cycle of an irrigation routine. Such an irrigation cycle may be sufficient for cleaning one ear of a human user, for example. Accordingly, such a disposable cleaning agent reservoir 700 may be changed out after every use, thereby reducing the number of components of the irrigation device 100 exposed to multiple users. In some implementations, the disposable cleaning agent reservoir 700 may be mounted directly onto the over-ear earpiece 101 using one or more fasteners or physical couplers. In some implementations, as discussed below, the disposable cleaning agent reservoir 700 may be loaded into a shell and mounted on the over-ear earpiece 101 when the shell is attached to the over-ear earpiece.

Figure 7B is an isometric view of a disposable discharge collection reservoir of an over-ear earpiece 720, according to at least one illustrated implementation not according to the invention. The disposable discharge collection reservoir 720 may include a front face 722 and a back face 724. In some implementations, the front face 722 and the back face 724 may be substantially parallel to each other. In such implementations, the disposable discharge collection reservoir 720 may include the one or more side walls 726 that bridge the distance between the front face 722 and the back face 724. In some implementations, the front face 722 and the back face 724 may meet at an edge such that the back face 724 curves outward from the front face 722 and/or the front face 722 curves outward from the back face 722. In such an implementation, a cross-sectional area of the disposable discharge collection reservoir 720 may include an arc along one or both of the sides associated with the back face 724 and the front face 722.

The front face 722, the back face 724, and the side walls 726 (when present) may define a hollow space 728 for the disposable discharge collection reservoir 720, with each of the front face 722, the back face 724, and the side walls 726 delineating a boundary for the hollow space 728. In some implementations, the hollow space 728 may be accessible via a disposable discharge collection reservoir port 730 located within a first depression 732 located on the front face 722 of the disposable discharge collection reservoir 720. The disposable discharge collection reservoir port 730 may be sized and dimensioned to selectively, detachably, physically mate with the discharge fluidly communicative path 221 at an end opposite the cannula 105, *e.g.*, at the opening 214a of the discharge passage 214. When the disposable discharge collection reservoir port 730 is mated with the discharge fluidly communicative path 221, the disposable discharge collection reservoir 720 may be used to collect discharge during one or more irrigation procedures. In some implementations, the disposable discharge collection reservoir port 730 may be sealed with a flexible membrane during a manufacturing and/or assembly process. In some implementations, the discharge fluidly communicative path 221 (*e.g.*, the tip of the discharge passage 214) may puncture the membrane thereby providing access to the hollow space 728 of the disposable discharge collection reservoir 720 when the disposable discharge collection reservoir 720 is mounted onto an over-ear earpiece 101.

The front face 722 of the disposable discharge collection reservoir 720 may include a vacuum assembly port 734 that may provide access to the hollow space 728 of the disposable discharge collection reservoir 720. The vacuum assembly port 734 may be located within a second depression 736 on the front face 722 of the disposable discharge collection reservoir 720. The vacuum assembly port 734 may be sized and dimensioned to physically, fluidly couple with the vacuum assembly 207b when the disposable discharge collection reservoir 720 is mounted onto an over-ear earpiece 101. The vacuum assembly or vacuum pump 207b may be used to create a vacuum or area of low pressure (*i.e*., lower than ambient environmental pressure, e.g., lower than 1 atmosphere) within the disposable discharge collection reservoir 720 to facilitate the removal of the discharge from the auditory canal during and/or after an irrigation procedure.

The disposable discharge collection reservoir 720 may be made of any suitable plastic materials, such as, for example, plastic material that is approved for use in medical procedures. In some implementations, the hollow space 728 of the disposable discharge collection reservoir 720 may hold sufficient volume to contain discharge from at least one (1) irrigation cycle of an irrigation routine. Such an irrigation cycle may be sufficient for cleaning one ear of a human user, for example. Accordingly, such a disposable discharge collection reservoir 720 may be changed out after every use, thereby reducing the number of components of the irrigation device 100 exposed to multiple users. In some implementations, the disposable discharge collection reservoir 720 may be mounted directly onto the over-ear earpiece 101 using one or more fasteners or physical couplers. In some implementations, as discussed below, the disposable discharge collection reservoir 720 may be loaded into a shell and mounted on the over-ear earpiece 101 when the shell is attached to the over-ear earpiece.

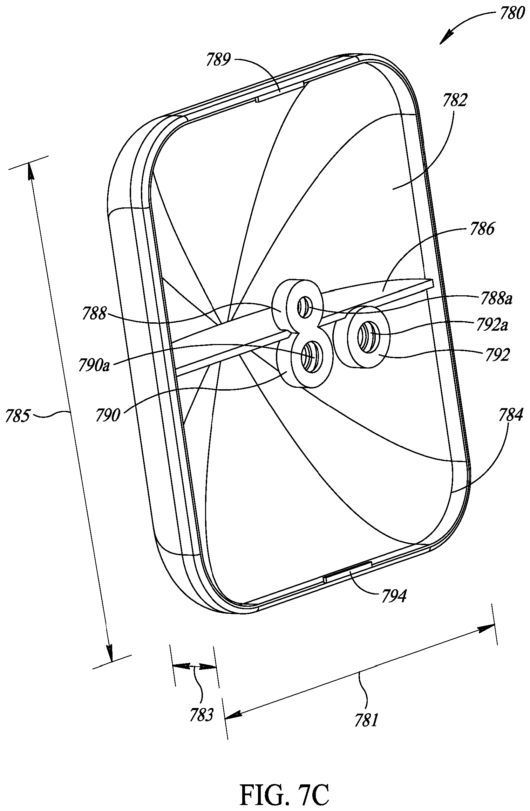

Figure 7C is an isometric view of a reservoir shell 780 broken into two reservoir cavities, a first cavity 782 which is sized and dimensioned to hold a disposable cleaning agent reservoir 700, and a second cavity 784 is sized and dimensioned to hold a disposable discharge collection reservoir 720, according to at least one illustrated implementation not according to the invention. The reservoir shell 780 may have a length 781, a width 783, and a height 785, and may be sized and dimensioned to fit over an exposed portion of an over-ear earpiece 101. The
reservoir shell 780 includes an interior wall 786 that runs across the length 781 of the reservoir shell 780 and separates the first cavity 782 from the second cavity 784. A first collar 788 may be attached to the interior wall 786 and extend perpendicularly to the interior wall 786 towards the first cavity 782. The first collar 788 may have an annular shape with an interior opening 788a. In some implementations, the interior opening 788a may align with the disposable cleaning agent reservoir port 710 when the disposable cleaning agent reservoir 700 is loaded into the first cavity 782. Such alignment may facilitate the mating of the disposable cleaning agent reservoir port 710 with the cleaning fluid fluidly communicative path 219.

In some implementations, the disposable cleaning agent reservoir 700 may be loaded into the first cavity 782 by sliding the end of the disposable cleaning agent reservoir 700 that has the disposable cleaning agent reservoir port 710 into the first cavity 782 towards the interior wall 786 and behind the first collar 788. In some implementations, the reservoir shell 780 may optionally have a latch 789 or other similar securing feature located along the edge of the first cavity 782 opposite the interior wall 786. The corresponding end of the disposable cleaning agent reservoir 700 may be pressed into first cavity 782 behind the latch 789 to thereby secure the disposable cleaning agent reservoir 700 into the first cavity 782.

A second collar 790 and a third collar 792 may be attached to the interior wall 786 and extend perpendicularly to the interior wall 786 towards the second cavity 784. The second collar 790 may have an annular shape with an interior opening 790a. In some implementations, the interior opening 790a may align with the disposable discharge collection reservoir port 730 when the disposable discharge collection reservoir 720 is loaded into the second cavity 784. Such alignment may facilitate the mating of the disposable discharge collection reservoir port 730 with the discharge fluidly communicative path 221. The third collar 792 may have an annular shape with an interior opening 792a. In some implementations, the interior opening 792a may align with an output from the vacuum assembly 207b when the disposable discharge collection reservoir 720 is loaded into the second cavity 784. Such alignment may facilitate the mating of the vacuum assembly port 772 with the vacuum assembly 207b.

In some implementations, the disposable discharge collection reservoir 720 may be loaded into the second cavity 784 by sliding the end of the disposable discharge collection reservoir 720 that has the disposable discharge collection reservoir port 730 into the second cavity 784 towards the interior wall 786 and behind the second collar 790 and/or third collar 792. In some implementations, the reservoir shell 780 may optionally have a latch 794 or other similar securing feature located along the edge of the second cavity 784 opposite the interior wall 786. The corresponding end of the disposable discharge collection reservoir 720 may be pressed into second cavity 784 behind the latch 794 to thereby secure the disposable discharge collection reservoir 720 into the second cavity 784.

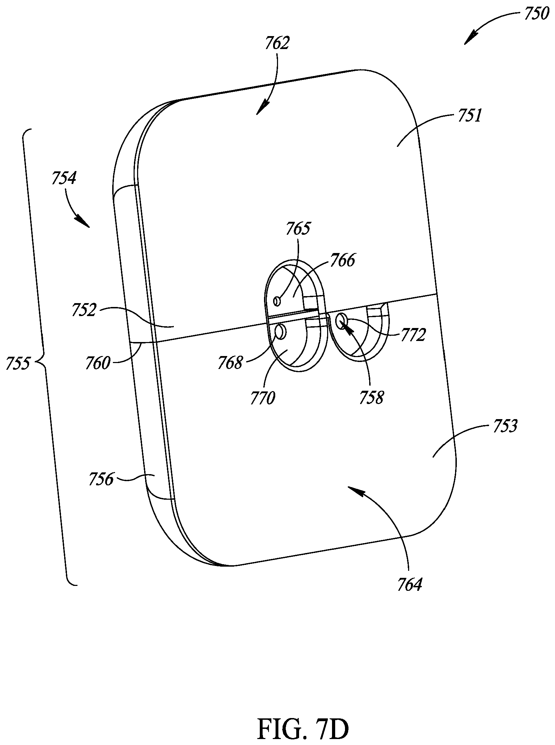

Figure 7D is an isometric view of a disposable container 750 that includes separate sections for a cleaning agent section 751 and a discharge collection section 753, according to at least one illustrated implementation not according to the invention. The disposable container 750 may include a front face 752 and a back face 754, and potentially one or more side walls 756, that form a unitary body 755. In some implementations, the front face 752 and the back face 754 may be substantially parallel to each other. In such implementations, disposable container 750 may include one or more side walls 756 that bridge the distance between the front face 752 and the back face 754. In some implementations, the front face 752 and the back face 754 may meet at an edge such that the back face 754 curves outward from the front face 752 and/or the front face 752 curves outward from the back face 752. In such an implementation, a cross-sectional area of the disposable container 750 may include an arc along one or both of the sides associated with the back face 754 and the front face 752.

The front face 752, the back face 754, and the side walls 756 (when present) may define a hollow space 758 for the disposable container 750, with each of the front face 752, the back space 754, and the side walls 756 delineating a boundary for the hollow space 758. In some implementations, a partition 760 may divide the hollow space 758 into multiple sections. In such implementations, the different sections may be fluidly separated from each other such that the fluid contents of one section would not be able to travel directly within the disposable container 750 to another section. For example, in some implementations, the hollow space 758 may be divided into a cleaning agent reservoir section 762 and a discharge collection reservoir section 764, with such sections fluidly separated from the other within the disposable container 750.

In some implementations, the cleaning agent reservoir section 762 of the hollow space 758 may be accessible via a cleaning agent port 765 located within a first depression 766 on the front face 702 of the disposable container 750. The cleaning agent port 765 may be sized and dimensioned to selectively, detachably, physically mate with the cleaning fluid fluidly communicative path 219 at an end opposite the cannula 105, *e.g.*, at the opening 212a of the cleaning agent passage 212. When the disposable container 750 is mated with the cleaning fluid fluidly communicative path 219, the cleaning agent reservoir section 762 may provide cleaning agent for one or more irrigation procedures. In some implementations, the cleaning agent port 765 may be sealed with a flexible membrane during a manufacturing and/or assembly process after cleaning agent has been placed into the cleaning agent reservoir section 762 of the disposable container 750. In some implementations, the cleaning fluid fluidly communicative path 219 (*e.g.*, the tip of the cleaning agent passage 212) may puncture the membrane thereby accessing the cleaning fluid when the disposable container 750 is mounted onto an over-ear earpiece 101.

In some implementations, the discharge collection reservoir section 764 may be accessible via a discharge collection reservoir port 768 located within a second depression 770 on the front face 752 of the disposable container 750. The discharge collection reservoir port 768 may be sized and dimensioned to selectively, detachably, physically mate with the discharge fluidly communicative path 221 at an end opposite the cannula 105, *e.g.*, at the opening 214a of the discharge passage 214. When the discharge collection reservoir port 768 is mated with the discharge fluidly communicative path 221, the discharge collection reservoir section 764 may be used to collect discharge during one or more irrigation procedures. In some implementations, the discharge collection reservoir section 764 may be sealed with a flexible membrane during a manufacturing and/or assembly process. In some implementations, the discharge fluidly communicative path 221 (*e.g.*, the tip of the discharge passage 214) may puncture the membrane thereby providing access to the discharge collection reservoir section 764 when the disposable container 750 is mounted onto an over-ear earpiece 101.

The front face 752 of the disposable container 750 may include a vacuum assembly port 772 that may provide access to the discharge collection reservoir section 764 of the disposable container 750. The vacuum assembly port 772 may be located within a third depression 774 on the front face 752 of the disposable container 750. The vacuum assembly port 772 may be sized and dimensioned to physically, fluidly couple with the vacuum assembly 207b when the disposable container 750 is mounted onto an over-ear earpiece 101. The vacuum assembly or vacuum pump 207b may be used to create a vacuum or area of low pressure (*i.e.,* lower than ambient environmental pressure, e.g., lower than 1 atmosphere) within the discharge collection reservoir section 764 to facilitate the removal of the discharge from the auditory canal during and/or after an irrigation procedure.

The disposable container 750 may be made of any suitable plastic materials, such as, for example, plastic material that is approved for use in medical procedures. In some implementations, the cleaning agent reservoir section 762 of the disposable container 750 may hold sufficient volume to contain discharge from at least one (1) irrigation cycle of an irrigation routine. In some implementations, the discharge collection reservoir section 764 may hold sufficient volume to contain discharge from at least one (1) irrigation cycle of an irrigation routine. Such an irrigation cycle may be sufficient for cleaning one ear of a human user, for example. Accordingly, such a disposable container 750 may be changed out after every use, thereby reducing the number of components of the irrigation device 100 exposed to multiple users. In some implementations, the disposable container 750 may be mounted directly onto the over-ear earpiece 101 using one or more fasteners or physical couplers. In some implementations, the disposable container 750 may be loaded into a shell (*e.g.,* reservoir shell 780 without interior wall 786) and mounted on the over-ear earpiece 101 when the shell is attached to the over-ear earpiece.

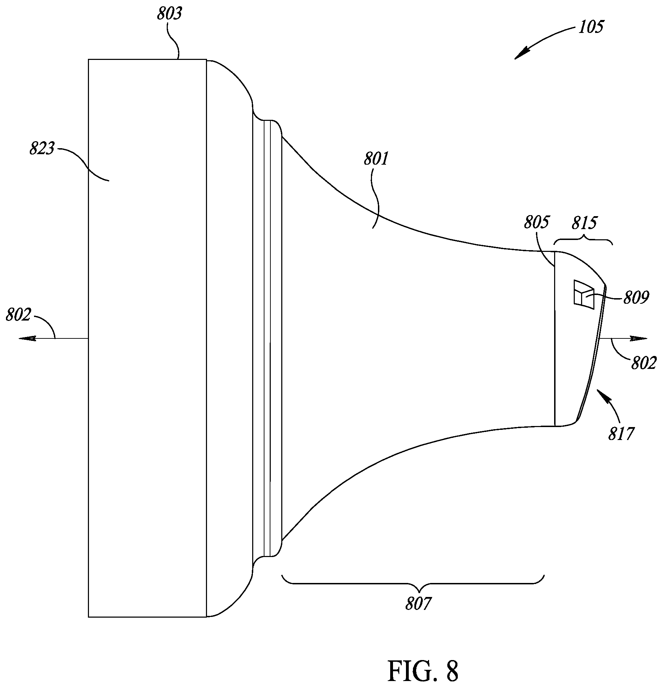

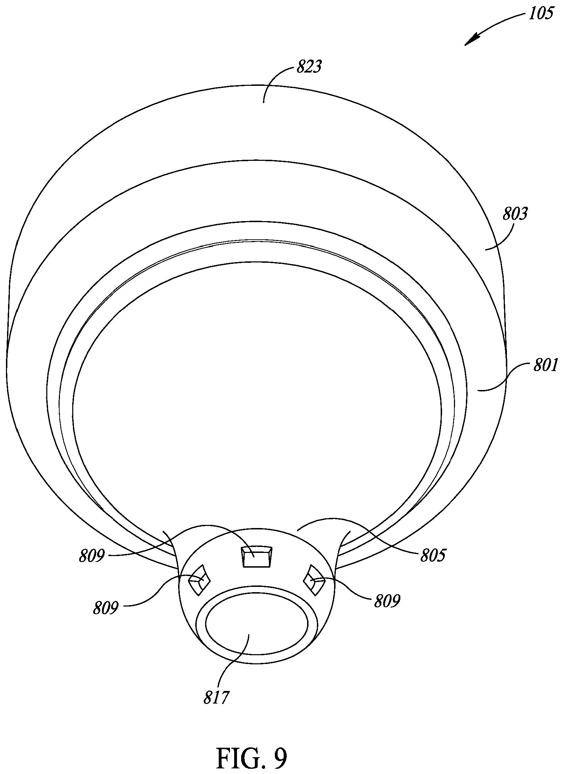

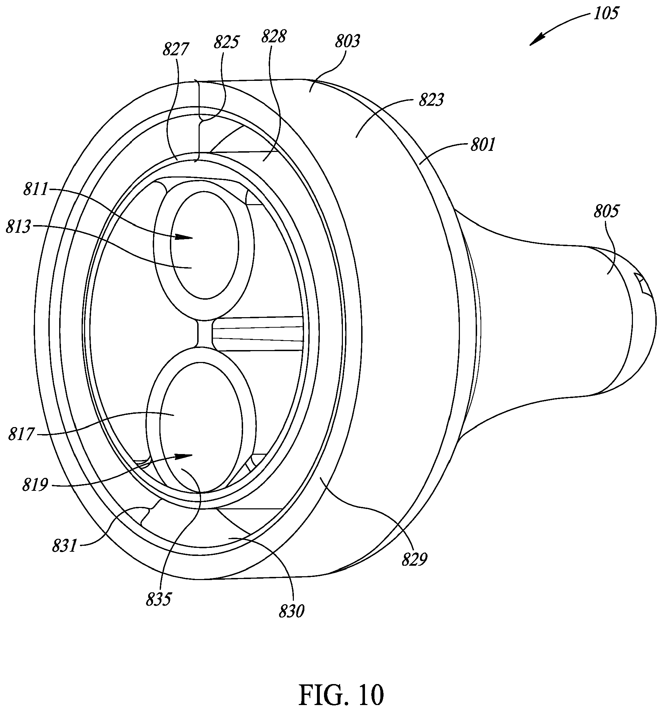

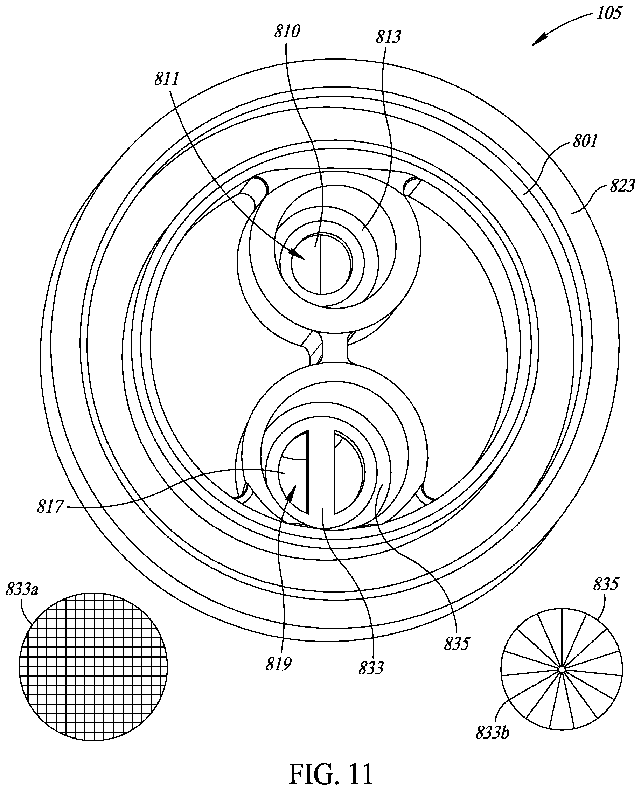

Figures 8, 9, 10, and 11 show a cannula 105 from various perspectives, according to one implementation. The cannula 105 includes a body 801 having a proximal end 803 and a distal end 805 separated by and opposing each other across a length 807. The body 801 may taper from the proximal end 803 towards the distal end 805, such that a cross-sectional area of the proximal end 803 is greater than a cross-sectional area of the distal end 805, which is inserted into the user's auditory canal. In such an implementation, the tapering of the cannula 105 may protect a user's ear drum by having a relatively larger portion of the body 801 impact a side wall of the user's auditory canal before the distal end 805 of the cannula 105 impacts the user's ear drum. In some implementations, the body 801 of the cannula 105 may have a partially conical shape, such as a frustro-conical shape in which the tapered end of the cone has been truncated. In some implementations, the tapering of the body 801 of the cannula 105 may be linear. In some implementations, the tapering of the body 801 of the cannula 105 may be non-linear, with a curve and/or steps in the profile as traversed from base to tip. In some implementations, the tapering may be linear over some portion of the body 801 of the cannula 105 and non-linear over other portions of the body 801 of the cannula 105. In some implementations, the body 801 of the cannula 105 may be formed of a unitary single-piece of plastic. In some implementations, alternatively, the body 801 of the cannula 105 may be formed of a plurality of pieces that have been joined together. In some implementations, the body 801 may have a form that is a body of rotation about a central axis 802. In some implementations, the body 801 of the cannula 105 may not be in the form of a body of rotation.

One or more irrigation outlet apertures 809 may be located proximate the distal end 805. In some implementations, one or more of the irrigation outlet apertures 809 may be set back a short distance (*e.g.,* up to 5 mm) from the edge at the distal end 805 of the cannula 105. The irrigation outlet apertures 809 may be used to direct an outward flow of cleaning agent that is exiting from the cannula 105. In some implementations, multiple irrigation outlet apertures 809 may be radially spaced around the body 801 of the cannula 105 proximate the distal end 805. The irrigation outlet aperture 809 may be located at the end of an irrigation passage 810 that provides an irrigation flow path 811 between an irrigation inlet port 813 located at the proximal end 803 of the cannula 105 and the irrigation outlet aperture 809 located relatively towards the distal end 805 with respect to the irrigation inlet port 813. The irrigation inlet port 813 may be substantially cylindrical in shape, or may have other shapes, for instance oval or elliptical.

A discharge collection inlet port 817 may be located at, or at least proximate, the distal end 805 of the cannula 105. The discharge collection inlet port 817 may be used to collect the discharge from the user's auditory canal during an irrigation procedure. In some implementations, a suction force may be created at the discharge collection inlet port 817 via a vacuum or area of relatively low or negative air pressure created by the vacuum assembly 207b within the discharge collection reservoir 205. The suction force may assist in collecting the discharge. The discharge collection inlet port 817 may be located at the end of a discharge flow path 819 that provides a flow path for the discharge between the discharge collection inlet port 817 located relatively towards the distal end 805 of the cannula 105 and the discharge collection outlet port 821 located at the proximal end 803 of the cannula 105. The discharge collection outlet port 821 may be substantially cylindrical in shape. In some implementations, the discharge collection outlet port 821 may take other shapes (*e.g.,* oval, elliptical, *etc*.).

In some implementations, the irrigation inlet port 813 is radially offset from the discharge collection outlet port 821. In some implementations, the irrigation outlet apertures 809 may be radially offset outwardly from the central axis 802; such placement may result in the irrigation outlet apertures 809 forming an arc. In some implementations, the discharge collection inlet port 817 may be disposed about the central axis 802.

A trap 833 may be located along the discharge flow path 819. In some implementations, for example, the trap 833 may be located proximate the proximal end 803 of the cannula 105. In some implementations, the trap 833 may be sized and dimensioned to trap physical debris of at least one defined dimension while passing at least one of a quantity of a liquid and air. For example, the trap 833 may be sized and dimensioned to trap ear wax being carried in the discharge. In some implementations, the trap 833 may be, for example, a filter 833a, for instance a mesh filter, woven filter, or non-woven filter, that extends across discharge flow path 819 and is oriented to be perpendicular to the direction of flow of the discharge in the discharge flow path 819. In some implementations, the trap 833 may include a plurality of fingers or projections 833b that extend radially inward from an inside perimeter 835 of the discharge collection passage 820. While a plurality of fingers or projections 833b are illustrated, the trap can comprise a single bar or rod or elongated member that extends all the way or at least partially across the discharge collection passage 820.

According to the claimed invention, the distal end 805 of the cannula 105 includes a beveled portion 815 that overhangs at least a portion of the discharge collection inlet port 817. In such implementations, the beveled portion 815 may position the discharge collection inlet port 817 such that it is directed at an angle with respect to the central axis 802, lying in a plane that is neither perpendicular nor parallel with the central axis 802. In some implementations, the beveled portion 815 may be shaped to orient the discharge collection inlet port 817 at a downward angle with respect to the horizontal axis 104 when a user in an upright position wears the irrigation device 100. Such an orientation may improve the effectiveness and efficiency of the suction introduced at the discharge collection inlet port 817 to collect the discharge of the irrigation procedure. In some implementations, the beveled portion 815 may include one or a plurality of the irrigation outlet apertures 809.

The proximal end 803 may include a flanged portion 823. In some implementations, the flanged portion 823 may include one or more interfaces to securely engage the cannula 105 to complementary interfaces on the cannula coupler interface 203, thereby securing the cannula 105 to the over-ear earpiece 101. In some implementations, the flanged portion 823 may include an interface 825 formed by an interior wall 827 that has an exterior diameter 828, an exterior wall 829 that has an interior diameter 830, and an open space 831 between the exterior diameter 828 and the interior diameter 830. In such an implementation, the cannula coupler interface 203 may include a corresponding, complementary interface 319, such as the annular wall 321, that is designed and shaped to fit tightly into the open space 831 and make contact with the exterior wall 829 and the interior wall 827. The cannula coupler interface 203 may securely hold the cannula 105 in place via frictional forces that will oppose movement of the cannula 105 when the annular wall 321 on the cannula coupler interface 203 is engaged between the interior wall 827 and the exterior wall 829. Such frictional forces, though, may not be so great as to prevent a user from removing the cannula 105 from the cannula coupler interface 203 when desired. Accordingly, such an interface 825 removably, securely engages the cannula 105 with the complementary interface 319 on the cannula coupler interface 203.

The flanged portion 823 may include other types of interfaces (*e.g.*, a threaded screw, a bayonet connector (*e.g.,* lugs and complementary recesses) that enable the cannula 105 to be removably, securely engaged with the cannula coupler interface 203. The flanged portion 823 may include one or more flexible, conformable materials (*e.g.,* rubber O-rings) that provide for a water tight and/or airtight seal between the cannula 105 and the cannula coupler interface 203.

The interface 825 may align the irrigation inlet port 813 located on the proximal end 803 of the cannula 105 with the cleaning agent port 316 located on the cannula coupler interface 203. The interface 825 may align the discharge collection outlet port 821 located on the proximal end 803 of the cannula 105 with the vacuum port 318 located on the cannula coupler interface 203. In such an implementation, when the cannula 105 is secured to the cannula coupler interface 203, the interface may provide that the irrigation inlet port 813 securely mates with the cleaning agent port 316, and that the discharge collection outlet port 821 securely mates with the vacuum port 318. One or more flexible, conformable seals may further be used to provide an air-tight and/or water-tight seal between irrigation inlet port 813 and the cleaning agent port 316, and/or the discharge collection outlet port 821 and the vacuum port 318.

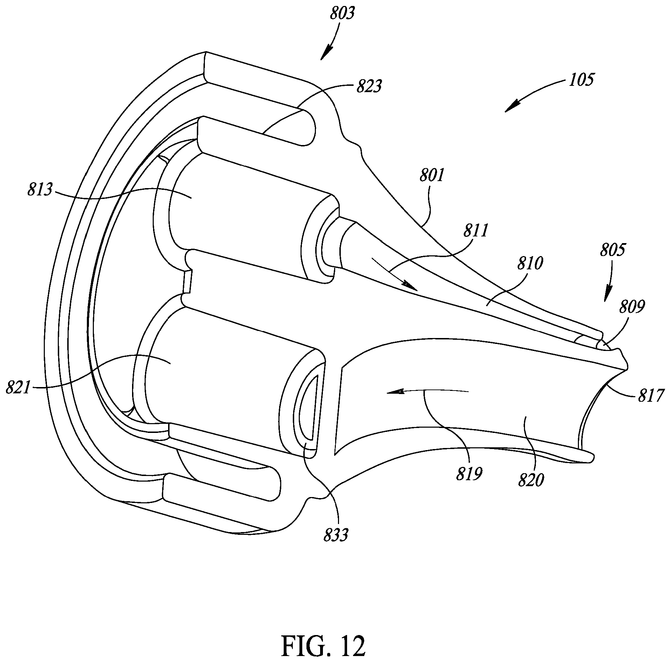

Figure 12 shows cannula 105 and better illustrates the paths of the irrigation passage 810 and the discharge collection passage 820, according to at least one illustrated implementation. The irrigation passage 810 connects the irrigation inlet port 813 located at the proximal end 803 of the cannula 105 with one or more irrigation outlet apertures 809 located proximate the distal end 805 of the cannula 105. The irrigation inlet port 813 may be substantially cylindrical, and sized and shaped to mate with the cleaning agent port 316 on the cannula coupler interface 203 when the cannula 105 is physically coupled with the cannula coupler interface 203. For example, the irrigation inlet port 813 may be a female connector or fastener, and the corresponding cleaning agent port 316 on the cannula coupler interface 203 may be a complementary male connector or fastener. The irrigation passage 810 may flatten as it progresses past the flanged portion 823 of the cannula 105 and goes towards the distal end 805 of the cannula. Such flattening may cause the irrigation passage 810 to form an arc shape that runs proximate the distal end 805 of the cannula 105 and extends between the various irrigation outlet apertures 809.

The discharge collection passage 820 provides a discharge flow path 819 between the discharge collection inlet port 817 and the discharge collection outlet port 821, and may be sized and shaped to mate with the vacuum port 318 on the cannula coupler interface 203 when the cannula 105 is physically coupled with the cannula coupler interface 203. For example, the discharge collection outlet port 821 may be a female connector or fastener, and the corresponding vacuum port 318 on the cannula coupler interface 203 may be a complementary male connector or fastener. The discharge collection passage 820 may have a smaller diameter at the distal end 805 of the cannula 105 compared to the diameter of the discharge collection passage 820 at the proximal end 803. Such narrowing may be gradual, such that the discharge collection passage 820 gradually tapers along the length of the cannula 105. Such narrowing may be abrupt and occur, for example, at a point where the discharge collection passage 820 moves out of the flanged portion 823 of the cannula. As discussed previously, a trap 833 may be located within the discharge collection passage 820, and sized, shaped, and oriented to capture particles of a specified size being carried within the discharge.

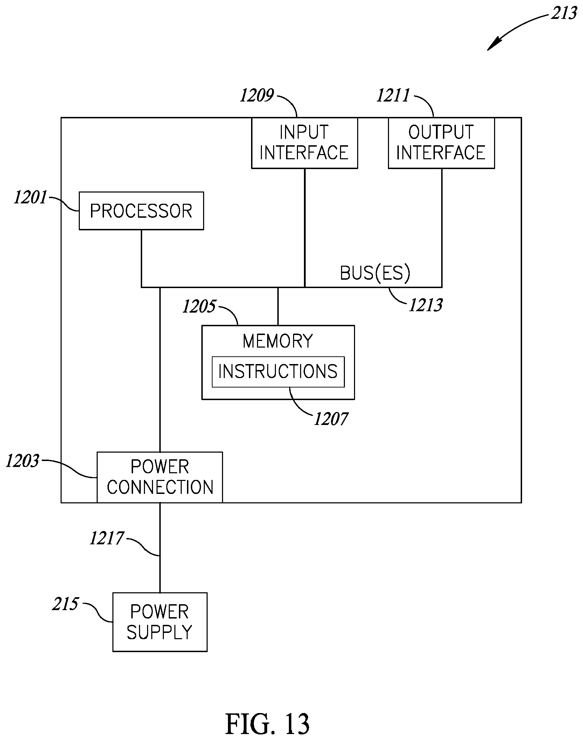

Figure 13 shows the controller circuitry 213 and the one or more power supplies 215, according to one illustrated implementation not in accordance with the invention. The controller circuitry 213 includes at least one processor 1201, a connection 1203 to a power supply 215 *(e.g.,* the one or more batteries), and one or more memories 1205 that store one or more sets of processor-executable instructions 1207, an input interface 1209 and an output interface 1211. Each of these components may be communicatively connected by bus(es) 1213, which can provide bidirectional communication between the various components of the controller circuitry 213. Bus(es) 1213 may take, for example, the form of a plurality of buses *(e.g.,* data buses, instruction buses, power buses) included in at least one body.

The input interface 1209 may be electrically and communicatively coupled to the user-actable selectable controls 217 and be used to receive user inputs in the form of electrical signals. Such user inputs may include, for example, selecting between a plurality of irrigation programs stored as sets of processor-executable instructions 1207 by the one or more memories 1205. The output interface 1211 may be electrically and communicatively coupled to one or both of the pump assembly 207a and the vacuum assembly 207b. The output interface 1211 may be used to transmit electrical signals generated by the processor 1201 and that result in activating or deactivating the pump assembly 207a and/or vacuum assembly 207b.

The processor 1201 may be any logic processing unit, such as one or more central processing units (CPUs), digital signal processors (DSPs), application-specific integrated circuits (ASICs), field programmable gate arrays (FPGAs), etc. The power supply 215 may include one or more power supplies 215, which provide electrical power to the various components of the irrigation device 100 via power connections 1217. The power supplies 215 may be an internal power supply, such as a battery, energy source, fuel cell, or the like.

The one or more memories 1205 may include read-only memory ("ROM") and random access memory ("RAM"). The one or more memories 1205 may comprise a flash drive to store data and/or processor-executable instructions. In some implementations, the one or more memories 1205 may include a hard disk drive for reading from and writing to a hard disk, an optical disk drive for reading from and writing to removable optical disks, and/or a magnetic disk drive for reading from and writing to magnetic disks. The one or more memories 1205 may communicate with the processor 1201 via the system bus 1213. Those skilled in the relevant art will appreciate that other types of computer-readable media that can store data accessible by a computer may be employed, such as WORM drives, RAID drives, magnetic cassettes, flash memory cards, digital video disks ("DVD"), Bernoulli cartridges, RAMs, ROMs, smart cards, etc.

The one or more sets of processor-executable instructions 1207, when executed, cause the irrigation device 100 to perform one or more irrigation routines. Such irrigation routines, when executed, may cause the processor 1201 to transmit a first signal to the vacuum assembly 207b via the output interface 1211 at a specified time that results in the vacuum assembly 207b being turned on, thereby creating a vacuum force to cause discharge to move into the discharge collection inlet port 817 of the cannula 105 through the discharge fluidly communicative path 221 and into the discharge reservoir 205. Such irrigation routines, when executed, may cause the processor 1201 to transmit a second signal to the pump assembly 207a via the output interface 1211 at a specified time that results in the pump assembly 207a being turned on, thereby forcing cleaning agent to move from or exit the cleaning agent reservoir 201 through the cleaning fluid fluidly communicative path 219 to the cannula 105, where it exits through the irrigation outlet apertures 809 at pressure. The processor 1201 may activate the pump assembly 207a using a plurality of pulses in which the pump assembly 207a is activated and deactivated for short time durations (e.g., about two seconds) during the irrigation routine before the pump assembly 207a is turned off. The set of processor-executable instructions 1207 may provide for irrigation procedures of different time periods (*e.g.,* up to 30 seconds, 30 seconds, one minute, or more than one minute).

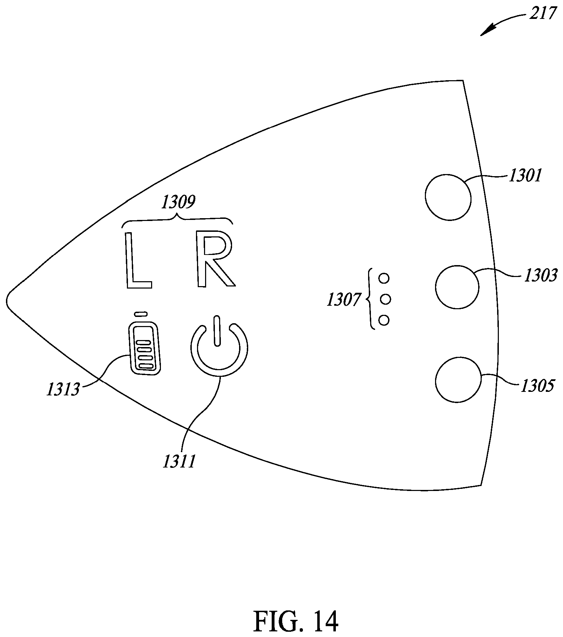

Figure 14 shows the user-actable selectable controls 217, according to at least one illustrated implementation not in accordance with the invention. The user-actable selectable controls 217 include an ear selection button 1301 and a program selection button 1303, a start button 1305, a set of LEDs 1307, an ear activation indicator 1309, a power indicator 1311, and a charging indicator 1313. The ear selection button 1301 may be used to turn on the irrigation device 100 and select the ear to be cleaned. In some implementations, the ear selection may be performed by touching a touch-sensitive or touch-responsive (e.g., resistance, inductive or capacitance touch sensors) area of the irrigation device 100 over or proximate the desired ear (*e.g.,* on or around junctions 135a and 135b). The program selection button 1303 may be used to select between a plurality of irrigation routines that may correspond to a plurality of sets of process-executable instructions 1207 stored within the one or more memories 1205. For example, the program selection button 1303 may be used to toggle or cycle through the plurality of irrigation routines. The set of LEDs 1307 may be used to show which routine is currently selected by the user. Thus, in some implementations, each LED may correspond to a location within the memory 1205 that stores instructions 1207 for a specific irrigation routine. The LED corresponding to the currently selected irrigation routine may be lit up. The start button 1305 may be used to start the currently selected irrigation routine.

In some implementations, the ear activation indicator 1309 may indicate which over-ear earpiece 101 is currently in operation, with the "R" corresponding to the over-ear earpiece 101a designed to fit over the user's right ear and the "L" corresponding to the over-ear earpiece 101b designed to fit over the user's left ear. The power indicator 1311 indicates whether the irrigation device 100 is currently powered on and running a cleaning cycle. The charging indicator 1313 indicates whether the irrigation device 100 is currently electrically coupled to a power source such that the battery 215 is being charged. The amount of charge that the battery 215 currently holds may be directly related to the amount of the charging indicator 1313 that is lit up. In addition, or alternatively, a fully charged battery 215 may cause the charging indicator 1313 to switch from one color (*e.g.,* red) that corresponds to the battery charging to a second color (*e.g.,* green) that indicates that the battery is fully charged. Some implementations may include other signal and indicators. For example, one or more indicators may be used to signify that the pump assembly 207a is providing cleaning agent at too high of a pressure, that the vacuum assembly 207b is providing too strong of a vacuum, that the discharge passage 214 is blocked, *etc.* In some instances, the irrigation device 100 may automatically shutdown to prevent injury to the user or damage to the irrigation device 100. In some implementations, at least one of the user-actable selectable controls 217 may be used as a "kill" switch to automatically shut down the irrigation device 100.

In some implementations, the irrigation device 100 may include touch screen controls to control the operation of and provide feedback regarding the irrigation device 100. Such touch screen controls may be incorporated into a portion of the irrigation device 100. In some implementations, the irrigation device 100 may have wireless communication capabilities (*e.g.*, via Bluetooth^{®}, WiFi^{®}, near field communications) such that the touch screen controls may be displayed via a wireless device, such as the user's smartphone/tablet or a dedicated tablet device).

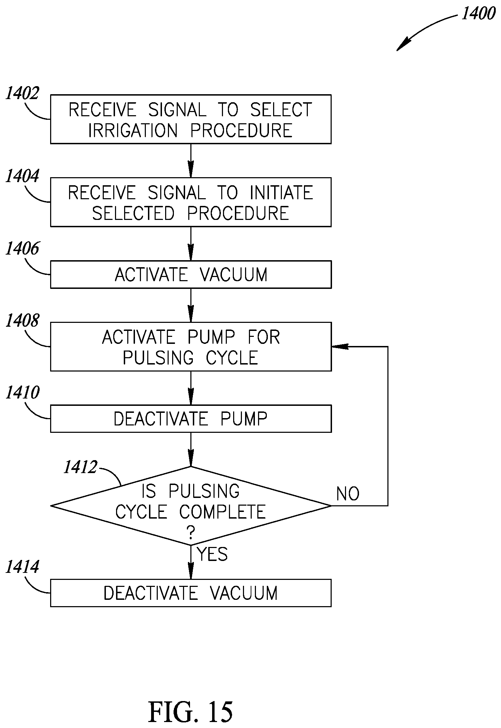

Figure 15 shows a method of operation 1400 of the irrigation device to perform an irrigation procedure, according to one illustrated implementation not in accordance with the invention. At 1402, a signal is received that selects between the various irrigation procedures stored within the one or more memories 1205 of the controller circuitry 213.

At 1404, a signal is received that initiates the currently selected irrigation procedure.

At 1406, vacuum assembly 207b is activated via a signal generated by controller circuitry 213 and transmitted through the output interface 1211.

At 1408, the pump assembly 207a is activated for a pulsing cycle. As noted before, activating the pump assembly 207a results in cleaning agent being dispensed from the cleaning agent reservoir 201 via the cleaning fluid fluidly communicative path 219 towards the cannula 105, where the cleaning agent exits under pressure. Thus, sending the signal to activate the pump assembly 207a causes a certain quantity of cleaning agent to be dispensed from the cleaning agent reservoir 201.

At 1410, the pump assembly 207a is deactivated after a time-delay is observed, thus providing one pulse of cleaning agent. The time delay and resulting pulse duration may be up to 2 seconds, for example. In some implementations, the time delay may be more or less than 2 seconds.

At 1412, a determination is made if the pulse cycle is complete. If the pulse cycle is not complete, then the operation 1400 goes to 1408 to activate the pump assembly 207a. In some implementations, each successive pulse may serve to soften and dislodge ear wax within the user's auditory canal. Each pulse cycle may be, for example, up to 30 seconds, one minute, 90 seconds, or longer. If the pulse cycle is complete, then the operation 1400 proceeds to 1414.

At 1414, the vacuum assembly 207b is deactivated to end the irrigation procedure.

The foregoing detailed description has set forth various implementations of the devices and/or processes via the use of block diagrams, schematics, and examples. Insofar as such block diagrams, schematics, and examples contain one or more functions and/or operations, it will be understood by those skilled in the art that each function and/or operation within such block diagrams, flowcharts, or examples can be implemented, individually and/or collectively, by a wide range of hardware, software, firmware, or virtually any combination thereof. Those of skill in the art will recognize that many of the methods or algorithms set out herein may employ additional acts, may omit some acts, and/or may execute acts in a different order than specified.

The various implementations described above can be combined to provide further implementations. In addition, all of the U.S. patents, U.S. patent application publications, U.S. patent applications, foreign patents, foreign patent applications and non-patent publications referred to in this specification and/or listed in the Application Data Sheet, including U.S. provisional patent application Serial No. 62/357,320, filed June 30, 2016 are mentioned.

Changes can be made to the implementations in light of the above-detailed description.

The claims are not limited by the disclosure The invention is defined by the features of the appended claims.

## Claims

1. A cannula, comprising:
a body (801) having a length, a proximal end (803), and a distal end (805), the distal end (805) opposite the proximal end (803) across the length of the body (801), the body (801) tapering from the proximal end (803) toward the distal end (805) and sized and shaped such that it can be inserted into an auditory canal, the body (801) positionable within one human ear such that the body (801) impacts a side wall of an auditory canal of the ear before the distal end (805) impacts an ear drum of the ear;
wherein the body (801) includes:
an irrigation inlet port (813) positioned at the proximal end (803);
a discharge collection outlet port (821) positioned at the proximal end (803);
a plurality of irrigation outlet apertures (809) positioned relatively toward the distal end (805) with respect to the irrigation inlet port (813);
a discharge collection inlet port (817) positioned at the distal end (805);at least one irrigation passage (810) that provides at least one irrigation flow path (811) between the irrigation inlet port (813) and the plurality of irrigation outlet apertures (809); and
at least one discharge collection passage (820) that provides at least one discharge flow path (819) that extends between the discharge collection inlet port (817) and the discharge collection outlet port (821); and
**characterized in that**
the distal end of the body includes a bevelled portion (815) that overhangs the discharge collection inlet port (817) and that includes the plurality of irrigation outlet apertures (809).

2. The cannula of claim 1, further comprising an interface configured to be removably coupleable to a complementary interface of an over-ear earpiece such that the irrigation inlet port of the body is aligned with a cleaning agent port of the over-ear earpiece and the discharge collection outlet port is aligned with a vacuum port of the over-ear earpiece.

3. The cannula of claim 1 or claim 2, further comprising at least one trap positioned in the discharge collection passage between the discharge collection inlet port and the discharge collection outlet port in the at least one discharge flow path.

4. The cannula of claim 3, wherein the at least one trap is a filter that extends at least partially across the discharge collection passage.

5. The cannula of claim 3 or claim 4, wherein the at least one trap is configured to trap physical debris of at least one defined dimension while passing at least one of a quantity of liquid and air.

6. The cannula of any preceding claim, wherein the plurality of irrigation outlet apertures comprises at least three irrigation outlet apertures proximate the distal end.

7. The cannula of any preceding claim, wherein at least one of the plurality of irrigation outlet apertures is configured to direct a flow of cleaning agent that exits the at least one of the plurality of irrigation outlet apertures radially outward from the distal end of the body.

8. The cannula of any preceding claim, wherein the cross-sectional area of the distal end is less than the cross-sectional area of the proximal end.

9. The cannula of any preceding claim, wherein the proximal end includes a flanged portion including one or more flexible, conformable materials that provide for a water tight or airtight seal between the cannula and a cannula coupler interface of an over-ear earpiece.

10. The cannula of any preceding claim, wherein the body is a unitary single-piece plastic body.

11. The cannula of any preceding claim, wherein the irrigation inlet port is radially offset from the discharge collection outlet port.

12. The cannula of any preceding claim, wherein the discharge collection inlet port is disposed about a longitudinal axis of the body.

13. The cannula of any preceding claim, wherein the discharge collection inlet port is directed at an angle with respect to a longitudinal axis of the body, lying in a plane that is neither perpendicular nor parallel with the longitudinal axis.

## Patentansprüche

1. Kanüle, umfassend:
einen Körper (801) mit einer Länge, einem proximalen Ende (803) und einem distalen Ende (805), wobei das distale Ende (805) dem proximalen Ende (803) über die Länge des Körpers (801) gegenüberliegt, wobei sich der Körper (801) von dem proximalen Ende (803) zu dem distalen Ende (805) hin verjüngt und so bemessen und geformt ist, dass er in einen Gehörgang eingesetzt werden kann, wobei der Körper (801) derart in einem menschlichen Ohr positionierbar ist, dass der Körper (801) auf eine Seitenwand eines Gehörgangs des Ohres wirkt, bevor das distale Ende (805) auf das Trommelfell des Ohres wirkt;
wobei der Körper (801) aufweist:
eine Spülungseinlassöffnung (813), die an dem proximalen Ende (803) positioniert ist;
eine Ausflusssammelauslassöffnung (821), die an dem proximalen Ende (803) positioniert ist;
eine Vielzahl von Spülungsauslassöffnungen (809), die bezogen auf das distale Ende (805) mit Bezug auf die Spülungseinlassöffnung (813) positioniert sind;
eine Ausflusssammelauslassöffnung (817), die an dem distalen Ende (805) positioniert ist; mindestens ein Spülungsdurchgang (810), der mindestens einen Spülungsfließweg (811) zwischen der Spülungseinlassöffnung (813) und der Vielzahl von Spülungsauslassöffnungen (809) bereitstellt; und
mindestens einen Ausflusssammeldurchgang (820), der mindestens einen Ausflussfließweg (819) bereitstellt, der sich zwischen der Ausflusssammeleinlassöffnung (817) und der Ausflusssammelauslassöffnung (821) erstreckt; und
**dadurch gekennzeichnet, dass** das distale Ende des Körpers einen abgeschrägten Abschnitt (815) aufweist, der die Ausflusssammeleinlassöffnung (817) überragt und der die Vielzahl von Spülungsauslassöffnungen (809) aufweist.

2. Kanüle nach Anspruch 1, ferner umfassend eine Schnittstelle, die dazu ausgelegt ist, derart entfernbar mit einer komplementären Schnittstelle eines Kopfhörers koppelbar zu sein, dass die Spülungseinlassöffnung des Körpers auf eine Reinigungsmittelöffnung des Kopfhörers ausgerichtet ist, und die Ausflusssammelauslassöffnung auf eine Vakuumöffnung des Kopfhörers ausgerichtet ist.

3. Kanüle nach Anspruch 1 oder Anspruch 2, ferner umfassend mindestens eine Falle, die in dem Ausflusssammeldurchgang zwischen der Ausflusssammeleinlassöffnung und der Ausflusssammelauslassöffnung in dem mindestens einen Ausflussfließweg positioniert ist.

4. Kanüle nach Anspruch 3, wobei die mindestens eine Falle ein Filter ist, der sich mindestens teilweise über den Ausflusssammeldurchgang erstreckt.

5. Kanüle nach Anspruch 3 oder Anspruch 4, wobei die mindestens eine Falle dazu ausgelegt ist, physischen Schmutz von mindestens einer definierten Abmessung einzufangen und dabei mindestens eins von einer Menge an Flüssigkeit und Luft durchzulassen.

6. Kanüle nach einem vorhergehenden Anspruch, wobei die Vielzahl von Spülungsauslassöffnungen mindestens drei Spülungsauslassöffnungen in der Nähe des distalen Endes umfasst.

7. Kanüle nach einem vorhergehenden Anspruch, wobei mindestens eine der Vielzahl von Spülungsauslassöffnungen dazu ausgelegt ist, einen Reinigungsmittelstrom, der aus der mindestens einen der Vielzahl von Spülungsauslassöffnungen austritt, von dem distalen Ende des Körpers radial nach außen zu lenken.

8. Kanüle nach einem vorhergehenden Anspruch, wobei die Querschnittsfläche des distalen Endes kleiner ist als die Querschnittsfläche des proximalen Endes.

9. Kanüle nach einem vorhergehenden Anspruch, wobei das proximale Ende einen geflanschten Abschnitt aufweist, der ein oder mehrere flexible, anpassbare Materialien aufweist, die für eine wasserdichte und luftdichte Abdichtung zwischen der Kanüle und einer Kanülenkopplerschnittstelle eines Kopfhörers sorgt.

10. Kanüle nach einem vorhergehenden Anspruch, wobei der Körper ein unitärer einstückiger Kunststoffkörper ist.

11. Kanüle nach einem vorhergehenden Anspruch, wobei die Spülungseinlassöffnung von der Ausflusssammelauslassöffnung radial versetzt ist.

12. Kanüle nach einem vorhergehenden Anspruch, wobei die Ausflusssammeleinlassöffnung um eine Längsachse des Körpers angeordnet ist.

13. Kanüle nach einem vorhergehenden Anspruch, wobei die Ausflusssammeleinlassöffnung in einem Winkel mit Bezug auf eine Längsachse des Körpers ausgerichtet ist und in einer Ebene liegt, die weder senkrecht noch parallel zu der Längsachse verläuft.

## Revendications

1. Canule, comprenant :
un corps (801) ayant une longueur, une extrémité proximale (803), et une extrémité distale (805), l'extrémité distale (805) étant en regard de l'extrémité proximale (803) sur la longueur du corps (801), le corps (801) s'effilant depuis l'extrémité proximale (803) vers l'extrémité distale (805) et étant dimensionné et formé de telle sorte qu'il puisse être inséré dans un conduit auditif, le corps (801) pouvant être positionné à l'intérieur d'une oreille humaine de telle sorte que le corps (801) heurte une paroi latérale du conduit auditif de l'oreille avant que l'extrémité distale (805) ne heurte le tympan de l'oreille ;
dans lequel le corps (801) comporte :
un orifice d'entrée d'irrigation (813) positionné au niveau de l'extrémité proximale (803) ;
un orifice de sortie de collecte d'évacuation (821) positionné au niveau de l'extrémité proximale (803) ;
une pluralité d'ouvertures de sortie d'irrigation (809) positionnées relativement vers l'extrémité distale (805) par rapport à l'orifice d'entrée d'irrigation (813) ;
un orifice d'entrée de collecte d'évacuation (817) positionné au niveau de l'extrémité distale (805) ; au moins un passage d'irrigation (810) qui fournit au moins un chemin d'écoulement d'irrigation (811) entre l'orifice d'entrée d'irrigation (813) et la pluralité d'ouvertures de sortie d'irrigation (809) ; et
au moins un passage de collecte d'évacuation (820) qui fournit au moins un chemin d'écoulement d'évacuation (819) qui s'étend entre l'orifice d'entrée de collecte d'évacuation (817) et l'orifice de sortie de collecte d'évacuation (821) ; et
**caractérisée en ce que** l'extrémité distale du corps comporte une partie biseautée (815) qui surplombe l'orifice d'entrée de collecte d'évacuation (817) et qui comporte la pluralité d'ouvertures de sortie d'irrigation (809).

2. Canule selon la revendication 1, comprenant en outre une interface configurée pour pouvoir être couplée de manière amovible à une interface complémentaire d'un écouteur supra-auriculaire de telle sorte que l'orifice d'entrée d'irrigation du corps soit aligné sur un orifice d'agent nettoyant de l'écouteur supra-auriculaire et que l'orifice de sortie de collecte d'évacuation soit aligné sur un orifice de vide de l'écouteur supra-auriculaire.

3. Canule selon la revendication 1 ou la revendication 2, comprenant en outre au moins un piège positionné dans le passage de collecte d'évacuation entre l'orifice d'entrée de collecte d'évacuation et l'orifice de sortie de collecte d'évacuation dans l'au moins un chemin d'écoulement d'évacuation.

4. Canule selon la revendication 3, dans laquelle l'au moins un piège est un filtre qui s'étend au moins partiellement à travers le passage de collecte d'évacuation.

5. Canule selon la revendication 3 ou la revendication 4, dans laquelle l'au moins un piège est conçu pour piéger des débris physiques d'au moins une dimension définie tout en laissant passer une quantité de liquide et/ou d'air.

6. Canule selon une quelconque revendication précédente, dans laquelle la pluralité d'ouvertures de sortie d'irrigation comprend au moins trois ouvertures de sortie d'irrigation proches de l'extrémité distale.

7. Canule selon une quelconque revendication précédente, dans laquelle au moins une ouverture de la pluralité d'ouvertures de sortie d'irrigation est conçue pour diriger un écoulement d'agent nettoyant qui sort d'au moins une ouverture de la pluralité d'ouvertures de sortie d'irrigation radialement vers l'extérieur à partir de l'extrémité distale du corps.

8. Canule selon une quelconque revendication précédente, dans laquelle la section transversale de l'extrémité distale est inférieure à la section transversale de l'extrémité proximale.

9. Canule selon une quelconque revendication précédente, dans laquelle l'extrémité proximale comporte une partie à bride comportant un ou plusieurs matériaux flexibles conformables qui assurent une étanchéité à l'eau ou à l'air entre la canule et une interface de raccord de canule d'un écouteur supra-auriculaire.

10. Canule selon une quelconque revendication précédente, dans laquelle le corps est un corps en plastique monobloc unitaire.

11. Canule selon une quelconque revendication précédente, dans laquelle l'orifice d'entrée d'irrigation est décalé radialement de l'orifice de sortie de collecte d'évacuation.

12. Canule selon une quelconque revendication précédente, dans laquelle l'orifice d'entrée de collecte d'évacuation est disposé autour d'un axe longitudinal du corps.

13. Canule selon une quelconque revendication précédente, dans laquelle l'orifice d'entrée de collecte d'évacuation est dirigé selon un angle par rapport à un axe longitudinal du corps, situé dans un plan qui n'est ni perpendiculaire ni parallèle à l'axe longitudinal.
